# EUROPÄISCHE PATENTSCHRIFT

(11) **EP 2 561 820 B1**
(45) Veröffentlichungstag und Bekanntmachung des Hinweises auf die Patenterteilung: **22.07.2020**
(21) Anmeldenummer: 11006973.9
(22) Anmeldetag: 26.08.2011
(51) Int. Cl.: B65D 83/26, A47K 5/12, B05B 9/04, B05B 12/12, A61B 90/80, B65D 83/38, B05B 9/043

(54) **Berührungslose Fluid-Applikationsvorrichtung**
Contactless fluid application device
Dispositif d'application de fluide sans contact

(43) Veröffentlichungstag der Anmeldung: 27.02.2013
(73) Patentinhaber: Schneider, Hartmut J., 67126 Hochdorf-Assenheim (DE); Prisman GmbH, 64653 Lorsch (DE)
(72) Erfinder: Schneider, Hartmut J., 67126 Hochdorf-Assenheim (DE); Metzger, Stephan, 64625 Bensheim (DE)
(74) Vertreter: Patentanwälte Dr. Keller, Schwertfeger Partnerschaft mbB

(56) Entgegenhaltungen:
- EP-A2- 2 228 774
- WO-A1-03/005873
- WO-A1-2006/134314
- WO-A2-2004/054460
- DE-A1-102004 063 782
- DE-U1-202007 012 093
- US-A- 3 419 188
- US-A1- 2002 074 349
- US-A1- 2004 217 137
- US-A1- 2004 226 962
- US-A1- 2007 000 941
- US-A1- 2008 185 399
- US-B1- 6 551 739

## Beschreibung

Die Erfindung betrifft eine berührungslos arbeitende Fluid-Applikationsvorrichtung, die insbesondere zur Verbesserung der Hygiene geeignet ist.

Gerade auch im medizinischen Bereich ist Hygiene ein besonders relevantes Thema: Noch immer infizieren sich pro Jahr in Deutschland 400.000 bis 600.000 Menschen während eines Aufenthaltes in einem Krankenhaus mit pathogenen Erregern, bis zu 30.000 Menschen sterben an den Folgen dieser teilweise sehr schweren Infektionen. Es wird davon ausgegangen, dass bis zu 90 % dieser nosokomialen Infektionen über die Hände übertragen werden. Aus diesem Grunde gehört die hygienische Handdesinfektion zu den wichtigsten Maßnahmen der Prophylaxe nosokomialer Infektionen im Besonderen und bzw. Infektionskrankheiten allgemein. Die Zielsetzung der Handdesinfektion ist in erster Linie die Verhinderung der Weiterverbreitung von auf die Hand gelangten Krankheitserregern.

Die Übertragung von Infektionskrankheiten (Bakterien oder Viren) erfolgt vorwiegend über Tröpfcheninfektion oder Schmierinfektion. Bei beiden Übertragungswegen spielen die Hände jedoch eine entscheidende Rolle. Handdesinfektion betrifft also nicht nur den medizinischen Sektor wie Krankenhäuser, Praxen und sonstige medizinische Einrichtungen, sondern auch alle Bereiche, in denen Menschen direkt oder indirekt miteinander in Berührung kommen.

Aufgrund der in letzter Zeit aufgetretenen Infektionskrankheiten wie etwa der Schweinegrippe und der Vogelgrippe und deren Präsenz in den Medien ist ein Wandel in der Wahrnehmung der Menschen bezüglich hygienischer Maßnahmen zu beobachten: Die Handdesinfektion hat den Sprung aus dem Bereich des Krankenhauses in den Endkonsumerbereich geschafft und wird als geeignete Vorsorgemaßnahme von einem Großteil der Bevölkerung wahrgenommen.

Handdesinfektionsmittel werden im Allgemeinen in Spenderflaschen im freien Handel und Fachhandel angeboten. Die Applikation des Handdesinfektionsmittels auf die Hand erfolgt über mechanische Dosierer oder einfach durch Aufträufeln des Mittels auf die Hand. Dabei werden ca. 3 ml des Mittels auf die Handinnenfläche gegeben und sollen dann über eine vom Hersteller vorgegebene Zeit (Einwirkzeit) zwischen den Händen und Fingern verrieben werden. Problematisch hierbei ist die teilweise unzureichende Benetzung der Haut, z. B zwischen den Fingern und unter den Fingernägeln durch den Anwender.

Dieses Problem wird durch automatisierte Spendersysteme minimiert, bei denen die Applikation durch automatisches Aufsprühen des Präparates erfolgt. Der Anwender hält beide Handinnenflächen so in Bezug zu einem Sensor einer automatisierten Spendervorrichtung, wonach der Sensor den Aussprühvorgang einer genau definierten Menge an Handdesinfektionsmittel auf beide Handinnenflächen des Anwenders initiiert.

Außer Desinfektionsmitteln sind jedoch auch Dosiervorrichtungen für andere Fluide wie Pflegemittel und Cremelotionen aus dem Stand der Technik hinreichend bekannt. Einfache Dosiervorrichtungen arbeiten dabei mit handbetätigbaren Pumpen und geben eine Dosis des Fluids nach einem Pumphub ab.

Einen Dosierspender, der es erlaubt, eine vorbestimmte Menge eines Fluids zu entnehmen, offenbart die DE 10 2009 008 022 A1: Dort wird ein Hygienefluid-Behälter mit einer Dosiervorrichtung ausgestattet, die manuell verstellbar ist und das dosierte Ausgeben des Hygienefluids erlaubt.

Weiter sind aus dem Stand der Technik verschiedene Dosiervorrichtungen bekannt, die von einem Gehäuse umgeben an einer Wand befestigt sein können oder in eine Fläche integriert sein können, die eine Waschvorrichtung umgibt. Gemeinsam sind diesen Dosierspendern Pumpvorrichtungen, die manuell aktiviert werden, meist mittels eines Hebels oder Druckknopfs, um eine mehr oder minder flüssige Substanz auszugeben. Die Pumpvorrichtung wird häufig als Kolbenpumpe realisiert. Einen weiteren Spender für Seife, Handlotion, Desinfektionsmittel oder dergleichen beschreibt die DE 35 05 893. Dabei erfolgt die Dosierung mittels einer Schlauchpumpe, wobei die Förderung der Flüssigkeit durch mechanische Quetschung des Schlauches mittels eines Griffs erfolgt.

Es gibt ferner Dosiervorrichtungen, die die berührungslose Ausgabe des Dosiergutes erlauben. Solche Vorrichtungen sind in der DE 35 31 385, der DE 101 03 890,der US 2008/0185399 A1 und DE 10 2004 063 782 A1 offenbart. Das berührungslose Ausgeben dient der Vermeidung der Keimübertragung bei der Aktivierung der Ausgabevorrichtung. Derartige berührungslose Vorrichtungen können sensorisch ausgelöst werden, insbesondere mittels optoelektronischer Sensoren, wie etwa die in der WO2004/054460 A3 offenbarte Vorrichtung zum Ausbringen von Desinfektions- und Reinigungsmitteln.

Ferner gibt es bereits Vorrichtungen zur Desinfektion, die es zusätzlich ermöglichen, sowohl Desinfektions- als auch Handreinigungsmittel sensorgesteuert auszubringen und zugleich Überschussflüssigkeit aufzufangen. Dazu beschreibt etwa die WO 2004/054460 A3 eine Vorrichtung zur Reinigung und/oder Desinfektion von Händen, die aus einem Gehäuse mit einem Tunnel besteht, an dessen Wandung ein Sensor und eine Sprühdüse zum Ausbringen von Flüssigkeiten angeordnet sind. Der Sensor löst eine mit der Düse verbundene Pumpe aus, die mit einem Vorratsbehälter verbunden ist. Der Tunnel weist einen zur Rückwand abfallenden Boden auf, unter dem sich eine Lade zum Aufsammeln von der Tunnelwandung ablaufender Flüssigkeit befindet, in der zusätzlich ein Absorptionsmittel vorgelegt wird, um das Überschussmedium zu binden. Die von der Düse auszubringenden Mengen an Desinfektionsmittel sind vorgeschrieben und betragen etwa 1,5 ml, was für einfache Zwecke ausreicht.

Die US 2007/0000941 A1 beschäftigt sich mit einem berührungslos betätigbaren Seifenspender, der Seife mittels einer Pumpe zu einem Auslass befördert. Hierbei ist der Applikationskopf auf den Seifenbehälter aufgeschraubt.

In der US 2007/0074349 A1 ist ein Schaumspender offenbart, der eine unter Druck stehende Dose und eine Heizvorrichtung zum Erwärmen des in der Dose befindlichen Produkts umfasst. Die unter Druck stehende Dose und die Heizvorrichtung sind in einem Gehäuse untergebracht, wobei sich die manuell betätigbare Heizvorrichtung in einem Applikationskopf befindet, der oberhalb der Dose angeordnet ist.

Die WO 2003/005873 A1 beschreibt einen berührungslos betätigbaren Seifenspender, der einen in einem Gehäuse untergebrachten Behälter mit Seife und Treibmittel aufweist, wobei an der Unterseite des Gehäuses eine Fluidausgabe vorgesehen ist.

Aus der US 2004/0217137 A1 und der DE 20 2007 012 093 U1 sind motorbetriebene Spender bekannt, bei denen unterhalb des Produktbehälters eine Fluidausgabe vorgesehen ist.

Nachteile dieser Vorrichtungen können darin gesehen werden, dass diese alle inflexibel einsetzbar und hinsichtlich ihrer Ausgestaltung entweder an eine bestimmte Vorratsbehälterform gebunden sind, damit ein sensorbasierter Spenderkopf sicher aufmontiert werden kann; oder dass sie sehr einfach gestaltet sind, so dass ein schlichter Förderschlauch in ein beliebiges Vorratsgefäß geführt werden kann, wobei diese Ausführungsformen weniger sicher in der Handhabung und Anordnung sind und gegebenenfalls eine Gesamtanordnung bilden, die wenig ansprechend ist und insofern einen Anwender auch nicht zur Benutzung animiert.

Ausgehend von diesem Stand der Technik ist es wünschenswert, eine Dosiervorrichtung zur Applikation von Fluiden, insbesondere zur Desinfektion zu schaffen, die einerseits automatisiert betreibbar und andererseits flexibel verwendbar für verschiedene Vorrichtungsgefäße ist und die dabei dennoch eine zur Benutzung animierende Form aufweist.

Diese Aufgabe wird durch die Fluid-Applikationsvorrichtung mit den Merkmalen der unabhängigen Ansprüche 1 und 4 gelöst.

Bevorzugte Weiterbildungen werden durch die Unteransprüche beschrieben.

In einer ersten Ausführungsform bezieht sich die erfindungsgemäße Fluidapplikationsvorrichtung mit automatischer Fluidausgabe auf eine Vorrichtung, die wenigstens einen Applikationskopf umfasst, der mit einem oder mehreren Sensoren und einer Düse ausgestattet ist, durch die Fluid ausgegeben werden kann. Die Düse ist dabei mit einem Vorratsbehälter fluidisch verbunden. Der Applikationskopf weist an seiner Oberseite einen Ausgabearm auf, an dem nach unten weisend die Düse angeordnet ist. Der Vorratsbehälter ist in dieser Ausführung der Erfindung ein unter Überdruck stehendes Gefäß, wie eine Druckdose, das, beziehungsweise die, einen Auslass aufweist, der durch ein Ventil geöffnet und geschlossen werden kann. In dem Applikationskopf liegt eine Steuerungseinheit vor, um sensorgesteuert das Öffnen und Schließen des Ventils zu veranlassen. Das Ventil ist über ein Verbindungsstück mit der Düse verbunden, so dass über die Düse das Fluid ausgegeben werden kann, wenn durch den Sensor veranlasst das Öffnen des Ventils mit einem entsprechenden Öffnungsmittel veranlasst wird. Weiter ist an dem Applikationskopf ein Adapterstück zum Halten des Vorratsbehälters vorgesehen, das dazu dient, den Vorratsbehälter an dem Applikationskopf zu halten, und das verschiedene Vorratsbehälter halten kann.

Durch diese Ausführungsform der Erfindung ist es möglich, ohne Zufuhr von externer Energie zum Pumpen des Fluids aus dem Vorratsbehälter zur Düse die Fluidausgabe zu tätigen. Es ist hierbei lediglich eine äußerst geringe Energiemenge erforderlich, um den Sensor zu betreiben. Damit kann dieses Gerät grundsätzlich batteriebetrieben werden, es ist daher von einer Netzversorgung unabhängig einsetzbar.

In einer weiteren Ausführungsform kann die Fluid-Applikationsvorrichtung mit automatischer Fluidausgabe, die ebenfalls einen Applikationskopf umfasst, der einen Sensor und eine Düse zum Ausgeben von Fluid aufweist, so gestaltet sein, dass der Sensor mit einer mit der Düse verbundenen Pumpe, die an den Vorratsbehälter angeschlossen ist, operativ verbunden ist. Weiter hat dieser Applikationskopf an seiner Oberseite einen Ausgabearm, an dem nach unten weisend die Düse angeordnet ist, wobei in dieser Ausführungsform in dem Applikationskopf eine motorbetriebene und durch den Sensor steuerbare Pumpe mit einem Ein- und einem Ausgang für Fluid angeordnet ist. Die Pumpe ist über ein Ventil ausgangsseitig mittels einer Schlauch- oder Rohrverbindung und mit einem Verbindungsstutzen der Düse, respektive einem Anschlussstück, zur Ausgabe des Fluids verbunden. Weiter ist die Pumpe über einem Adapterstück angeordnet und kann mit diesem mit einem Vorratsgefäß fluiddicht verbunden werden.

Dabei kann das Adapterstück an dem Applikationskopf dauerhaft oder lösbar angeordnet sein. Dies erfolgt über die Unter- oder Innenseite des Adapterstücks, die mit einer Vorratsgefäß- Öffnung in Eingriff gebracht werden kann, so dass sich eine Fördervorrichtung, insbesondere ein Schlauch oder ein Rohr von der Eingangsseite der Pumpe in das Vorratsgefäß erstreckt.

Das Adapterstück kann so gestaltet sein, dass seine Oberseite formschlüssig mit dem Applikationskopf verbindbar ist und seine Unterseite einen Flansch aufweist, insbesondere einen Flansch, der von einem Rand des Adapterstücks nach innen versetzt ist und der mit der Öffnung des Vorratsgefäßes in Eingriff gebracht werden kann, indem der Flansch einen Vorratsgefäß-Hals umgreift oder in diesen eintaucht. Ein Förderschlauch erstreckt sich von der Eingangsseite der Pumpe durch eine in dem Adapterstück vorgesehene Öffnung in das Vorratsgefäß, um so das Fluid auf Aktivwerden des Sensors hin zu fördern.

Die Aktivierung des Sensors in den erfindungsgemäßen Ausführungsformen erfolgt durch das Hinführen eines Körperteils, wie insbesondere einer Hand oder alternativ eines Fußes, in den Erfassungsbereich des Sensors.

Die Sensoren, die für die erfindungsgemäße Fluid-Applikationsvorrichtung gemäß beiden Ausführungsformen besonders geeignet sind, sind optische oder optoelektronische Sensoren. Die Applikationsvorrichtung kann dabei mit mehreren Sensoren ausgestattet sein, wobei jedoch zumindest ein Sensor an dem Ausgabearm entweder nach unten weisend oder an dem Applikationskopf in Richtung der Düse weisend angeordnet ist, um so eine aktivierende Hand, die unter die Düse geführt wird, in jedem Fall zu detektieren. Um vorteilhaft eine Handform oder eine Fußform zu bestimmen, kann der Sensor Reflexion messen. Bei der Reflexionsmessung ist es sogar möglich, Falten in der Körperhaut wahrzunehmen und aus der Tiefe von Falten auf den Bedarf an Desinfektionsmittel oder an Pflegefluid rückzuschließen, das durch die Applikationsvorrichtung ausgegeben wird.

Es ist auch möglich, mehrere Sensoren an dem Ausgabearm und/oder der vertikalen Wandung des Applikationskopfes vorzusehen, um eine genauere Bestimmung der Größe und Körperteilform des mit Fluid zu versehenden Körperteils zu ermitteln.

In der Variation der Ausführungsform, bei der das Fluid gepumpt werden muss, bei der also der Sensor operativ mit einer Pumpe gekoppelt ist, so dass das Auslösen durch den Sensor den Pumpenantrieb tätig werden lässt, wird eine Membranpumpe bevorzugt, die ausgangsseitig mit einem Einwegventil operativ gekoppelt ist. Die Membranpumpe muss durch eine Energieversorgungseinheit versorgt werden, wobei es sich zwar um eine Netzversorgung mittels einer dem Fachmann bekannten Netzleitung und entsprechenden Bauteilen handeln kann, wobei jedoch bevorzugt ein in seiner Gesamtheit austauschbares Batteriemagazin vorzusehen ist, das entsprechend in elektrischem Kontakt mit der Pumpe steht. Durch die Gestaltung als Magazin oder Kartusche kann das lästige Austauschen einzelner Batterien entfallen und das gesamte Magazin kann vorteilhaft mit einem Griff aus der Fluid-Applikationsvorrichtung entnommen werden. Besonders vorteilhaft kann dazu der Applikationskopf geöffnet werden, beispielsweise durch das Vorsehen einer öffenbaren Kopfabdeckung.

Der eine oder die mehreren Sensoren ist/sind über eine Steuerungs- und/oder Steuerungs- und Regelungseinheit mit der Pumpe operativ gekoppelt, die auf einer Platine angeordnet sein kann. So ist es möglich, die Ausgabevolumina des Fluids zum einen voreinzustellen oder zum anderen abhängig von Form, Größe, Hautfaltentiefe oder anderen Regelungsparametern zu regeln.

Bei der Ausführungsform, bei der das Vorratsgefäß eine Druckdose ist, dient die Steuerungseinheit dem Steuern des Ventils, sie kann jedoch zusätzlich als Steuerungs- und Regelungseinheit ausgebildet sein, um ebenfalls in Abhängigkeit der Handgröße, Form und den weiteren genannten Parametern eine Ausgabemenge eines Fluids zu regeln. In Frage kommen hierbei abgestufte ml-Einteilungen von 0,5 in Halbmilliliterstufen aufsteigend bis etwa 3,0 ml, es ist jedoch auch möglich, hier die Ventilöffnungszeit stufenlos einzustellen. Ebenso kann, allerdings bei der Variante, bei der ein Pumpenhub getätigt wird, auch eine nahezu stufenlose Einstellung erfolgen.

Bei der Variante, bei der ein Förderschlauch die Pumpe mit dem Vorratsgefäß verbindet und wobei der Förderschlauch durch die Öffnung des Adapterstücks geführt wird, kann ein durchbohrter Stopfen, insbesondere ein Stopfen mit Doppellochführung in dem Adapterstück vorgesehen sein, durch den der Förderschlauch abdichtend in der Öffnung befestigt wird.

Vorteilhaft weist die Fluid-Applikationsvorrichtung, insbesondere in dem Applikationskopf angeordnet, eine Vorrichtung zur Dichtemessung des Fluid, und/oder eine Vorrichtung zur Hand- und Hautbeschaffenheitserkennung, wie bereits durch die Sensoren erläutert, oder alternativ oder zusätzlich eine Vorrichtung zur Handgrößenbestimmung auf, was ebenfalls durch die genannten Sensoren realisiert werden kann. Die Vorrichtungen zur Handgrößen- oder Hautbeschaffenheitsbestimmung sind operativ mit der Steuerungs- und/oder Regelungsvorrichtung für die Pumpe beziehungsweise für das Ventil gekoppelt.

Die erfindungsgemäße Fluid-Applikationsvorrichtung kann ferner auch so gestaltet sein, dass das Adapterstück als eine Überwurfmutter ausgebildet und mit dem Applikationskopf verbunden ist. Diese Gestaltung ist besonders deshalb praktisch, weil die Überwurfmutter quasi auf jede Flaschengröße mit Hals-Außengewinde geschraubt werden kann; ggf. kann das Adapterstück mit Überwurfmutter entsprechend ausgewählt und am Applikationskopf ausgetauscht werden. Platzsparend und ästhetisch ist die Fördervorrichtung dort ein doppelwandiges Förderrohr, insbesondere ein Koaxial-Förderrohr, dessen Innenrohr das Batteriemagazin bildet, das zur Energieversorgung zumindest mit der Pumpe, insbesondere mit der Pumpe und einem oder weiteren in dem Applikationskopf vorliegenden Energieverbrauchern elektrisch verbunden ist. Zusätzlich kann eine Vorratsflasche mit Desinfektionsmittel oder einem anderen Fluid, die mit dieser Applikationsvorrichtung ausgestattet ist, in eine Halterung zur Verbesserung der Standsicherheit eingesetzt werden.

Sowohl das Druckgefäßadapterstück für Druckdosen als auch das Adapterstück für nicht unter Druck stehende Flaschen oder Gefäße kann sich in ein Mantelstück, respektive ein Gehäuse erstrecken, das zumindest einen Abschnitt des Vorratsgefäßes umgibt. So wird die Vorrichtung optisch attraktiv und kann zur Anbringung oder Stabilisierung weitere Funktionen erfüllen oder Elemente zur Erfüllung von Aufgaben wie Stabilisierung oder Wandanbringung oder weiteres aufnehmen.

Das Mantelstück kann dabei bei einer zur Wandanordnung vorgesehenen Fluid-Applikationsvorrichtung so ausgebildet sein, dass es an seiner zur Wand weisenden Seite oder an der Bodenseite geöffnet ist, um so das Einführen des Vorratsbehälters zu erlauben. Die Wandbefestigung kann über eine Wandhalterung erfolgen. Im Übrigen kann das Mantelstück auch einstückig mit der Wandhalterung ausgebildet sein.

Weiter kann zur flexiblen Nutzung der Vorrichtung das Mantelstück oder die mit diesem einstückig ausgebildete Wandhalterung innenseitig eine Liftvorrichtung aufweisen, die aus einer Aufstandsfläche für das Vorratsgefäß und einem Schienenelement besteht, so dass die Aufstandsfläche einen vertikal verschiebbaren Boden an dem Mantelstück bildet. Damit können kleine Flaschen einfach auf die Aufstandsfläche gestellt und zu dem Adapterstück hingefahren werden.

Alternativ zur Wandbefestigung kann an der Wandhalterung eine Schraubklemme angebracht sein, so dass die Applikationsvorrichtung vorteilhaft an einem horizontalen Tragarm oder einem Möbel mit horizontal ausgerichteter Befestigungsfläche angeordnet sein kann.

Ferner kann, gerade bei einer Ausbildung der Fluid-Applikationsvorrichtung als Stand-Element, das Mantelstück sensorseitig beziehungsweise betätigungsseitig offen sein, so dass das Vorratsgefäß von vorne eingeführt beziehungsweise eingesteckt werden kann. Auch hier, bei der Variation als Stand-Bauteil, kann das Einführen eines Vorratsgefäßes von unten erfolgen, so dass auch die Möglichkeit besteht, das Mantelstück so zu gestalten, dass es das Vorratsgefäß umfänglich vollständig umgibt.

Dabei kann ein vorderer Abschnitt des Mantelstück der den zur Hand weisenden Umfangsabschnitt des Vorratsgefäßes verhüllt, gelenkig aufklappbar und über ein Scharnier, das an diesem Teil und dem unteren Rand des Mantelstücks vorliegt, angelenkt sein. Es kann statt der gelenkigen Anordnung auch durch eine Ausführung mit einem Schienenpaar mit jeweils korrespondierenden Elementen an den beiden Mantelstück- und vorderen Abschnittsrändern von unten aufgeschoben werden.

Das Mantelstück erstreckt sich bei einer Ausgestaltung als besonders formschöne Applikationsvorrichtung vorzugsweise bis an den unteren Rand des Vorratsgefäßes oder darüber hinaus. An dem unteren Rand des Mantelstückes kann eine Auffangtropfschale vorgesehen sein, die sich entsprechend der Ausdehnung des Ausgabearms unter diesen erstreckt, um überschüssiges Fluid aufzunehmen. Dies kann erforderlich sein, wenn der Bediener der Applikationsvorrichtung beispielsweise zwei Ausgabevorgänge auslöst und dabei feststellt, dass zu viel Fluid ausgegeben wird und die Hand wegzieht. Gerade im Fall von Desinfektionsmitteln, die alkoholisch oder aggressiv sein können und insofern zu lästigen und unerwünschten Dämpfen führen können, kann die Auffangtropfschale gute Dienste leisten.

In einer Ausführungsform kann die Auffangschale einen doppelten Boden aufweisen. Dabei hat der obere Boden mehrere Durchtrittsöffnungen, um das Fluid abfließen zu lassen, wobei die Durchtrittsöffnungen vorzugsweise am Rand der Auffangschale vorgesehen sein können. Am unteren Boden kann eine Absaugvorrichtung, wie beispielsweise ein zum Absaugen betriebener Rotor vorgesehen sein, der so in fluidischem Kontakt mit den Durchtrittsöffnungen steht, dass überschüssige Flüssigkeit unmittelbar durch die Durchtrittsöffnungen nach unten weggesaugt wird.

Vorteilhaft ist daher am oder im unteren Boden des doppel- oder mehrbödigen Elements ein oder mehrere Absorptionsmittel für Fluid angeordnet. Statt eines doppelten Bodens kann hier auch einfach eine Haltestruktur für ein Absorptionsmittel wie ein Pad angebracht sein. Hierbei kann es sich entweder um bekannte partikuläre Absorptionsmittel handeln, jedoch auch um ein Saugvlies oder ein Saugpad, abhängig davon, wie die chemische Beschaffenheit des auszugebenden Fluids ist.

Die Auffangtropfschale kann, was insbesondere zur Reinigung vorteilhaft ist, lösbar an dem Mantelstück angeordnet sein. Alternativ kann die Auffangtropfschale jedoch auch einstückig mit dem Mantelstück ausgebildet sein. In einer weiteren Ausführungsform weist die Auffangtropfschale an einer Anschlussstelle der Auffangtropfschale an das Mantelstück eine Durchführung für eine Energieversorgungsleitung auf, die erforderlich ist, wenn der Saugrotor mit einer Netzleitung und nicht durch Batteriebetrieb betätigt beziehungsweise versorgt wird. Selbstverständlich ist es auch hier denkbar, eine derartige Absaugvorrichtung energiearm und batteriebetrieben mit Energie zu versorgen, wobei es möglich ist, in dem Mantelstück eine Aufnahmevorrichtung für Batterien und eine entsprechende elektronische Kopplung zu der Saugvorrichtung vorzusehen. Auch hier können Batterien in Magazinen untergebracht werden, um so ein erleichtertes Austauschen zu ermöglichen.

In einer noch weiteren Ausführungsform kann der Antrieb der Absaugvorrichtung mit dem Sensor, der entweder die Ventilöffnung oder den Pumpenbetrieb auslöst, kommunikativ und operativ verbunden sein. Die Verbindung kann auch unter Zwischenschaltung der Steuerungs- und/oder Regelungseinheit erfolgen, so dass unmittelbar mit dem Auslösen der Pumpe oder dem Öffnen des Ventils und damit der Fluidausgabe auch die Absaugung in Gang gesetzt wird, um so zu vermeiden, dass beim Starten des Ausgabevorgangs zunächst überschüssiges Fluid, das gegebenenfalls auch von den Händen abprallt, in die Umgebungsluft austritt.

In einer Ausführungsform der Fluid-Applikationsvorrichtung, die als Vorratsbehälter ein Druckgefäß und einen Auslass mit Ventil verwendet, kann das Druckgefäßadapterstück so ausgestaltet sein, dass das Druckgefäß mittels einer Klemmhalterung hält. Die Klemmhalterung kann insbesondere derart ausgestaltet sein, dass wenigstens zwei jeweils mittels einer Klemmfeder vorgespannte Umgriffelemente, die in einem Rahmenteil radial verschiebbar angeordnet sind, Druck gegen das Gefäß ausüben und es insofern halten. Dabei können die Klemmfedern entweder außenseitig gegen einen speziellen Aufsatz abgestützt sein, in dem die Klemmhalterung angeordnet ist. Dieser Aufsatz kann in den Applikationskopf angedockt werden oder er kann zumindest teilweise in diesen eingesetzt werden und lässt somit Platz für das Mantelteil.

Es ist auch möglich, die Klemmfedern gegen eine Innenwandung des Applikationskopfes abgestützt anzuordnen. Zum vorteilhaften Betätigen und Lösen des Vorratsbehälters mittels des Druckgefäßadapterstücks ist eine Betätigungsvorrichtung wie eine Stellschraube zum Vorspannen und Lösen der Klemmfedern vorgesehen.

Diese und weitere Vorteile werden durch die nachfolgende Beschreibung unter Bezug auf die begleitenden Figuren dargelegt.

Der Bezug auf die Figuren in der Beschreibung dient der Unterstützung der Beschreibung und dem erleichterten Verständnis des Gegenstands. Gegenstände oder Teile von Gegenständen, die im Wesentlichen gleich oder ähnlich sind, können mit denselben Bezugszeichen versehen sein. Die Figuren sind lediglich schematische Darstellungen von Ausführungsbeispielen der Erfindung.

Es zeigt:

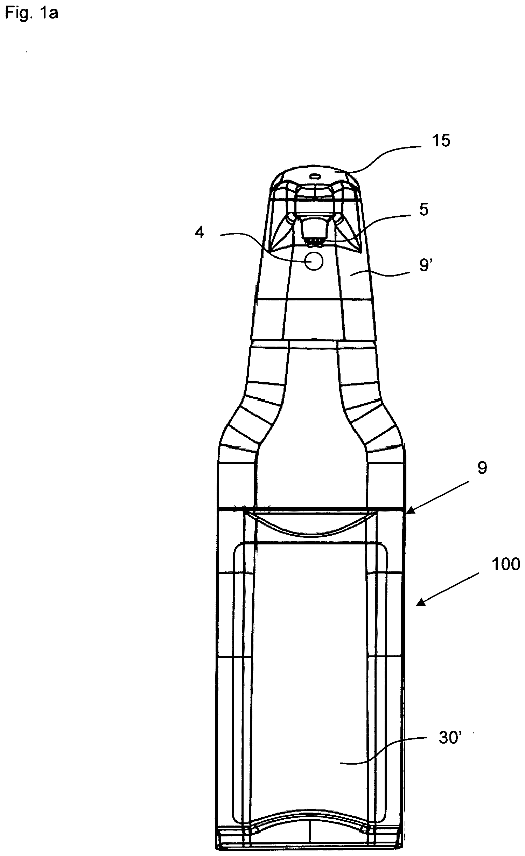

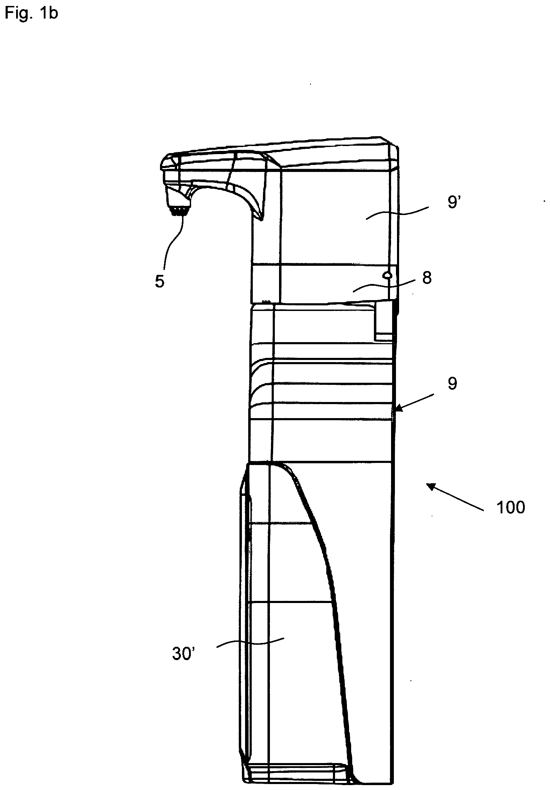

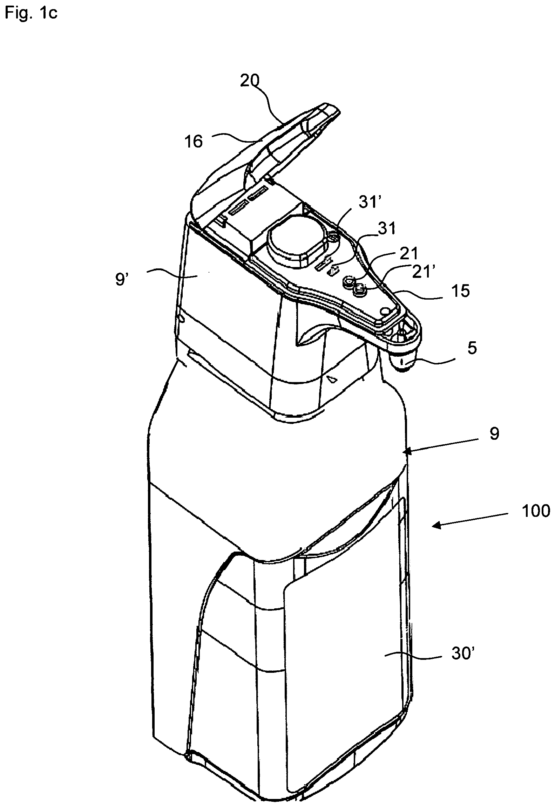

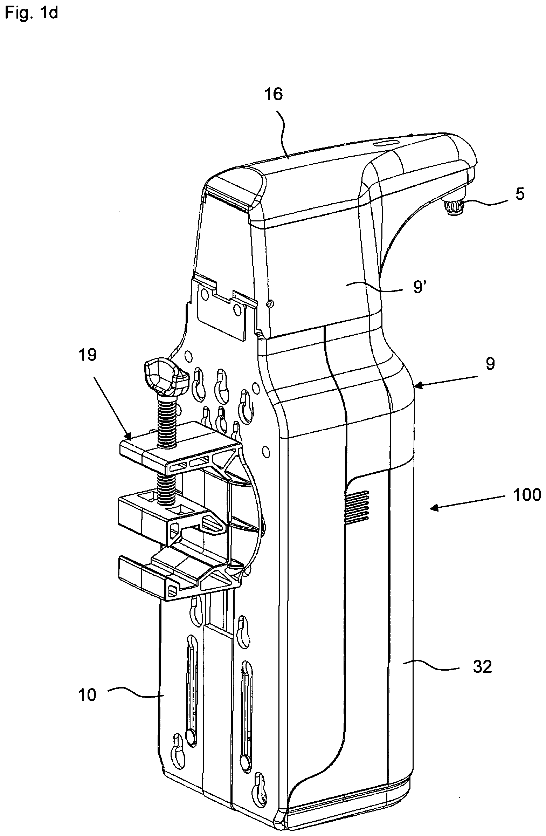

- Fig. 1 a :: eine Vorderansicht auf die Ausgabeseite der Fluid-Applikationsvorrichtung,
- Fig. 1 b :: eine Seitenansicht auf die Fluid-Applikationsvorrichtung, schmale Ausführung,
- Fig. 1 c :: eine perspektivische Ansicht einer Applikationsvorrichtung mit breitem Vorratsgefäß, bei geöffneter Kopfabdeckung,
- Fig. 1d :: eine perspektivische Ansicht einer Applikationsvorrichtung mit Schraubklemme zur Anbringung an einem Möbel,
- Fig. 2a :: eine Seitenansicht auf den Kopf der Fluid-Applikationsvorrichtung,
- Fig. 2b :: eine Vorderansicht auf die Ausgabeseite des Kopfes aus Fig. 2a,
- Fig. 2c :: eine Innenansicht in den Kopf der Fluid-Applikationsvorrichtung bei geöffneter Abdeckung des Applikationskopfes,
- Fig. 2d :: eine perspektivische Innenansicht in den Kopf der Fluid-Applikationsvorrichtung,
- Fig. 2e :: eine Draufsicht in das Innere des Kopfs der Fluid-Applikationsvorrichtung,
- Fig. 2f :: eine perspektivische Seitenansicht des Kopfs der Fluid-Applikationsvorrichtung mit einer Verbindung in ein Vorratsgefäß,
- Fig. 2g :: eine perspektivische Seitenansicht des Kopfs der Fluid-Applikationsvorrichtung mit einer Verbindung in ein Vorratsgefäß über ein Adapterstück,
- Fig. 3a :: eine Untenansicht auf die Fluid-Applikationsvorrichtung mit breitem Flaschenhaltegehäuse,
- Fig. 3b :: eine Ansicht von oben auf die Fluid-Applikationsvorrichtung, große Ausführung,
- Fig. 4 :: eine perspektivische Explosionsansicht von Komponenten, die den FluidApplikationskopf bilden, und ein Adapter zur Anbringung eines Fluidgefäßes an dem Applikationskopf,
- Fig. 5a-5j :: perspektivische Darstellungen der Komponenten aus Fig. 4 ,
- Fig. 6 :: eine perspektivische Ansicht einer integrierten Fluid-Applikationsvorrichtung mit Auffangschale für Druckdosen,
- Fig. 7a :: eine perspektivische Ansicht auf eine Desinfektionsmitteldose und einen Aufsatz zur Anordnung an einem Fluid-Applikationskopf,
- Fig. 7b :: eine perspektivische Ansicht des Aufsatzes mit Klemmhalterung aus Fig. 6a,
- Fig. 7c :: eine andere perspektivische Ansicht des Aufsatzes mit Klemmhalterung aus Fig. 6a,
- Fig. 7d :: die Halterung aus Fig. 6a,
- Fig. 7e :: eine Explosionsansicht der Klemmhalterung aus Fig. 6a, und des Aufsatzes,
- Fig. 8a :: eine Draufsicht auf eine Auffangschale zur Anordnung an einer erfindungsgemäßen Applikationsvorrichtung,
- Fig. 8b :: eine Seitenansicht der Auffangschale aus Fig. 7a , mit Wandhalterung,
- Fig. 9a :: eine Ansicht von vorn auf die Auffangschale mit Wandhalteelement zur Anordnung an der Applikationsvorrichtung,
- Fig. 9b :: eine Seitenansicht der Auffangschale mit Wandhalterung,
- Fig. 10 :: eine Explosionsansicht der Auffangschale mit Absaugungsrotor und eine perspektivische Draufsicht auf die Auffangschale,
- Fig. 11 :: eine Auffangschale mit Rotor von unten,
- Fig. 12 :: eine Draufsicht auf eine erfindungsgemäße Auffangschale mit Absaugungsrotor,
- Fig. 13a :: eine Vorderansicht an eine Wandhalteplatte zur Anordnung der FluidApplikationsvorrichtung an der Wand,
- Fig. 13b :: eine Seitenansicht der Wandhalterung aus Fig. 12a,
- Fig. 14 :: eine Applikationsvorrichtung mit aufklappbarem vorderen Mantelstück,
- Fig. 15a :: eine perspektivische Ansicht der Applikationsvorrichtung zur Flaschenaufschraubung mit geöffnetem Kopf,
- Fig. 15b :: eine perspektivische Ansicht der Applikationsvorrichtung zur Flaschenaufschraubung mit teilweise geöffnetem Kopf,
- Fig. 15c :: eine perspektivische Ansicht der Applikationsvorrichtung zur Flaschenaufschraubung mit geschlossenem Kopf,
- Fig. 15d :: eine Seitenschnittansicht der Applikationsvorrichtung zur Flaschenaufschraubung mit geschlossenem Kopf,
- Fig. 16 :: eine kombinierte Mantelstück-Wandhalterung mit Lift.

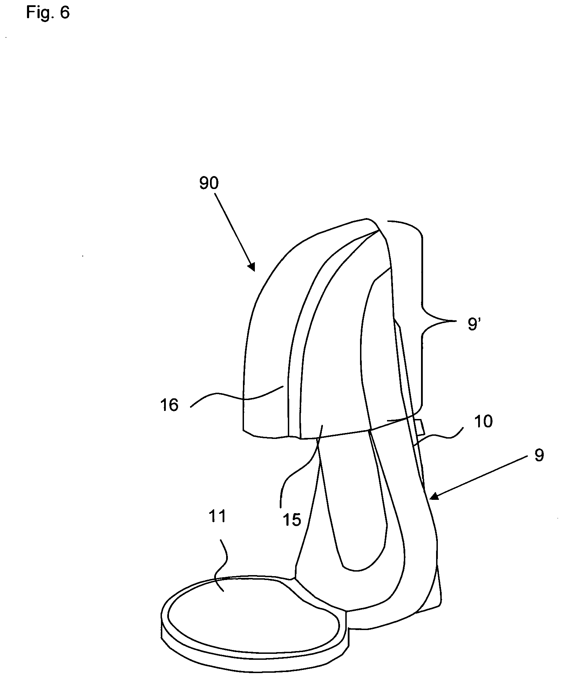

Die vorliegende Erfindung bezieht sich auf eine Fluid-Applikationsvorrichtung 90, wie sie in Fig. 6 gezeigt ist und die sich insbesondere zur Verwendung mit Druckbehältern wie Aerosol-Dosen 30 eignet, oder auf Fluid-Applikationsvorrichtungen, die sich unter Verwendung von Pumpen zum Fördern eines Desinfektions- oder Pflegemittels in flüssiger, Gel-, Emulsions- oder anderer Form eignen.

Derartige Ausführungsformen sind insbesondere in den Fig. 1a, b und c mit dem Bezugszeichen 100 gezeigt. Die erfindungsgemäßen Fluid-Applikationsvorrichtungen 90, 100, sind sämtlich zur automatischen Fluidausgabe geeignet und weisen einen Applikationskopf 9' auf, an dem ein Sensor 4 und eine Ausgabedüse 5 zum Ausgeben von Fluid angeordnet ist. Die Düse 5 ist dabei jeweils mit einem Vorratsbehälter 30 fluidisch verbunden.

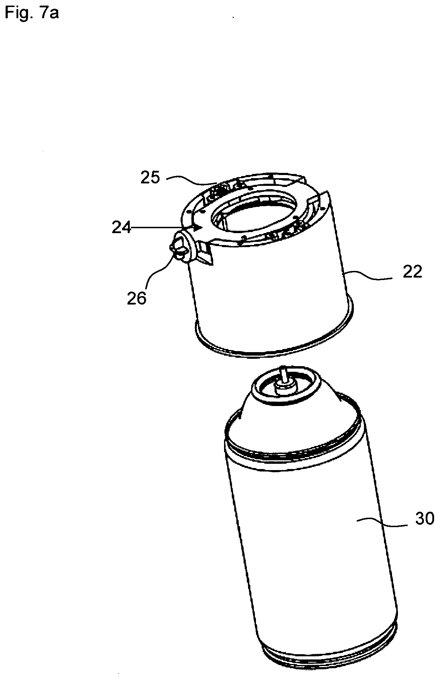

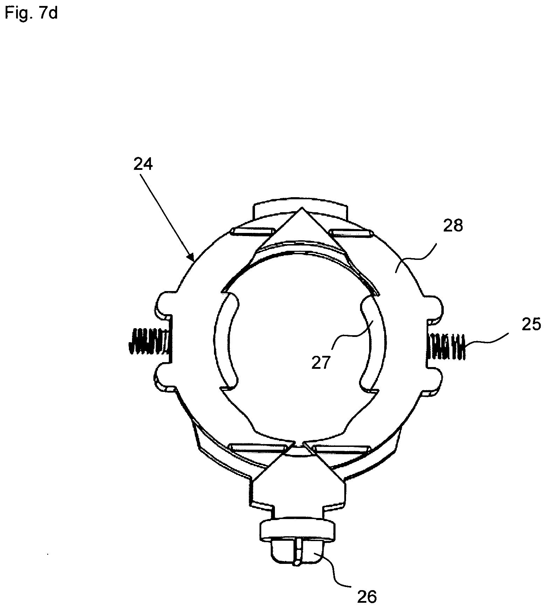

In der in Fig. 6 gezeigten Ausführungsform für Druckdosen weist der Applikationskopf 9' an seiner Oberseite einen integrierten Ausgabearm, nicht separat dargestellt, auf, an dem nach unten weisend die Düse, figurativ nicht dargestellt, angeordnet ist. Der Vorratsbehälter 30 ist eine unter Überdrück stehende Aerosoldose 30, wie sie auch in Fig. 7a gezeigt ist, so dass gemäß dieser Realisierung der Fluid-Applikationsvorrichtung auf Mittel zum Fördern des Fluids verzichtet werden kann. Die Abdeckung 16 des Applikationskopfes 9' kann durch Flansche an der Abdeckung 16 und an dem Kopf, die quasi eine Schienenführung bilden, hochgeschoben werden. Das Hochschieben kann auch automatisch und hydraulisch erfolgen. In diesem Falle ist ein kleiner Motor zum Ausführen der Verschiebung in dem Applikationskopf 9' vorzusehen.

In dem Applikationskopf 9' liegt jedoch eine Steuerungseinheit zum sensorgesteuerten Öffnen und Schließen des Ventils der unter Druck stehenden Aerosoldose 30 vor, das an der Oberseite der in Fig. 7a gezeigten Dose 30 zu erkennen ist. Das Ventil ist über ein Verbindungsstück mit der Düse zur Ausgabe des Fluids verbunden.

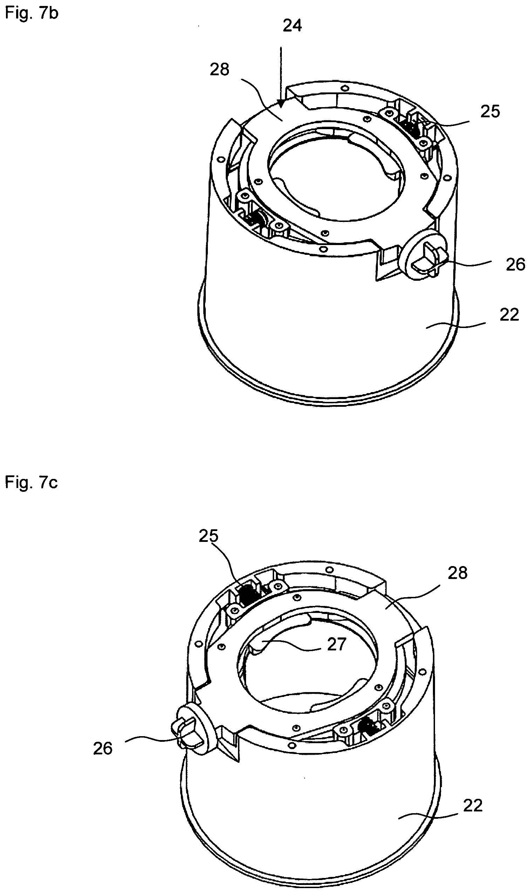

An dem Applikationskopf 9' ist intern ein Druckgefäßadapterstück angeordnet, das dem Halten des Druckgefäßes 30 in der Applikationsvorrichtung dient. Ein derartiges Druckgefäßadapterstück ist in einer einfachen Form in Fig. 7a bis 7d gezeigt: Es ist als eine Klemmhalterung ausgebildet, wobei die Klemmhalterung 24 zwei mittels jeweils einer Klemmfeder 25 vorgespannte Umgriffelemente 25' aufweist, die in dem Rahmenteil 28 radial verschiebbar angeordnet sind und somit den Kopf der Druckdose 8' einklemmen. Die Klemmfedern 25 können in dem Aufsatz 22 gemäß Fig. 7b und c abgestützt sein. Dort sind auch die Betätigungsvorrichtungen, hier als Stellschrauben 26 ausgebildet, zum Vorspannen und Lösen der Klemmfedern 25 gezeigt. Durch Drehen der Stellschraube kann somit der Durchmesser der Klemmvorrichtung verjüngt oder geweitet werden, je nach Größe der zu haltenden Druckdose, wodurch sich die Adapterfunktion ergibt.

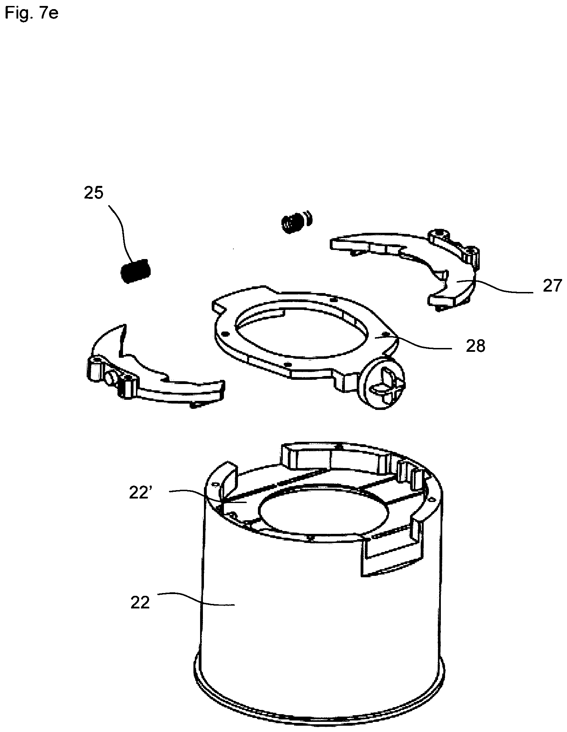

Fig. 7e zeigt die Klemmhalterung 24 mit den Klemmfedern 25 und dem Rahmenteil 28 in Explosionsansicht, zur Anordnung in dem Aufsatz 22, der zylindrisch ausgebildet ist und an seiner Oberseite eine Ausnehmung mit Abstützflansch 22' aufweist, auf den die Klemmhalterung 24 aufgesetzt werden kann. Alternativ kann die in Fig. 7b gezeigte Klemmhalterung jedoch auch direkt in den Adapterkopf 9' der Fluid-Applikationsvorrichtung, wie in Fig. 6 gezeigt, eingesetzt werden.

Die in Fig. 6 gezeigte Ausführungsform zeigt ferner ein Mantelstück 9, das quasi das "Gehäuse" der Applikationsvorrichtung bildet. Es ist in Richtung der Bedienseite geschlossen und hat jedoch eine zylindrische Auswölbung, an der innenseitig die Druckdose, die von unten eingeschoben werden kann, anliegt.

Zur Montage an der Wand ist an der rückwärtigen Seite der Applikationsvorrichtung 90 eine Wandhalterung 10 vorgesehen, hier ohne elektronische Durchführungen.

Alternativ kann die Applikationsvorrichtung 90, ebenso die Applikationsvorrichtung 100, mit der Schraubklemme 19, wie sie an der Wandhalterung 10 angebracht und in Fig. 1d gezeigt ist, zur Anbringung an einem Möbel wie einem Krankenbett oder -tisch vorgesehen sein, so dass vorteilhaft ein Behandler, der Kranke selbst oder Besucher jederzeit die Hände desinfizieren und so eine Keimübertragung verhindern können.

Weiter zeigt Fig. 6 eine Auffangschale 11, die unmittelbar an den Mantelabschnitt angeschnitten ist und dazu dient, überschüssig ausgegebenes Fluid aufzufangen. Sie kann in einer einfachen Ausführungsform doppelbödig mit eingelegtem Absorptionsmittel, beispielsweise mit Pads benutzt werden, die das überschüssige Fluid aufsaugen und die entsprechend ausgetauscht werden können, wenn das doppelbödige Auffangschalenelement 11 wie vorgeschlagen zum Aufklappen ausgestaltet ist oder zumindest das Herausziehen und Austauschen von Pads zulässt.

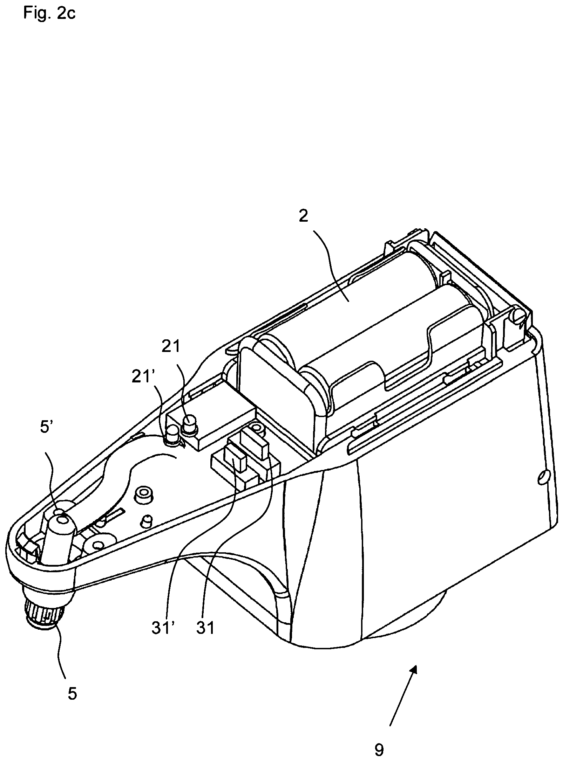

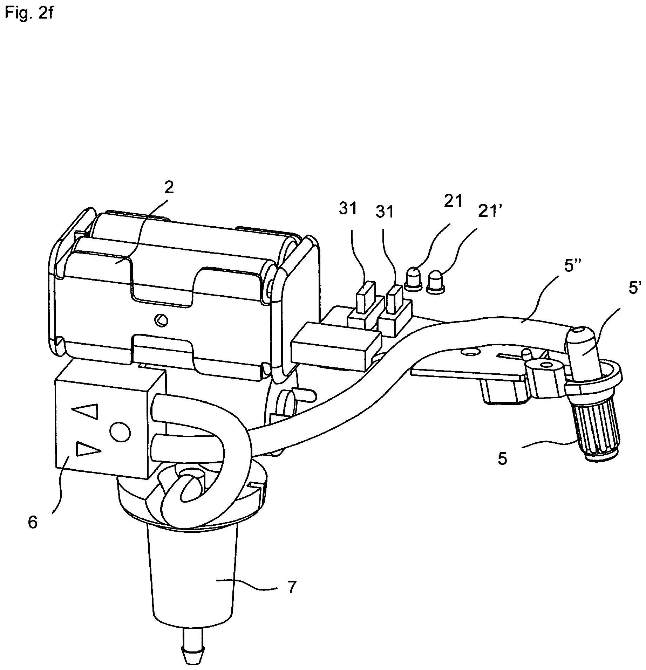

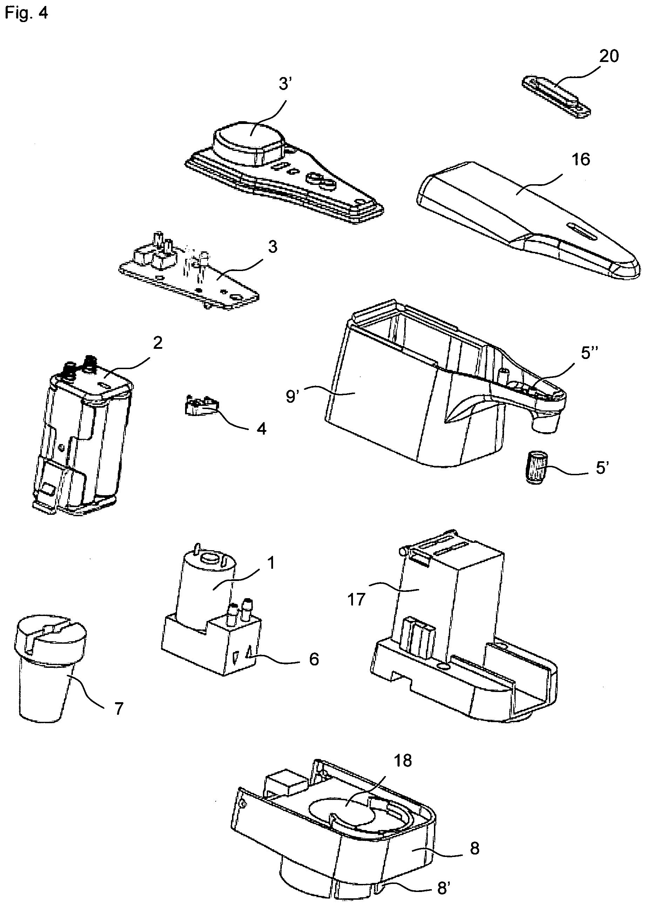

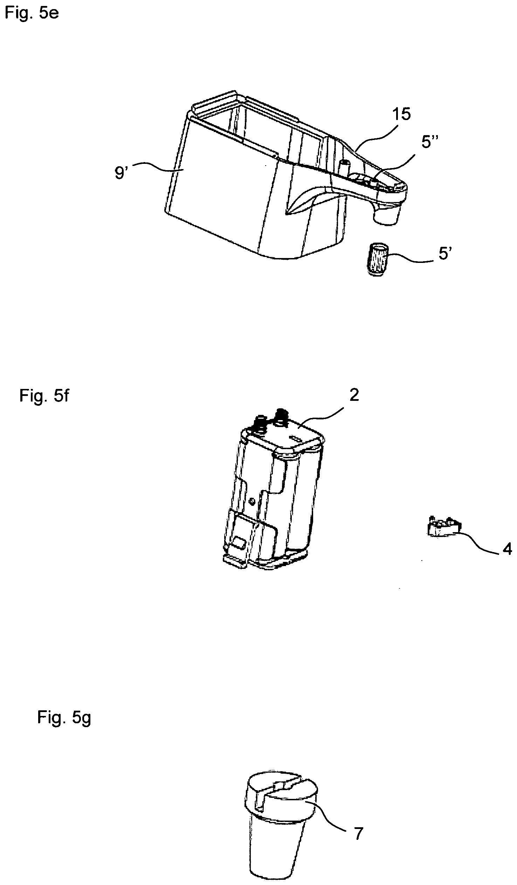

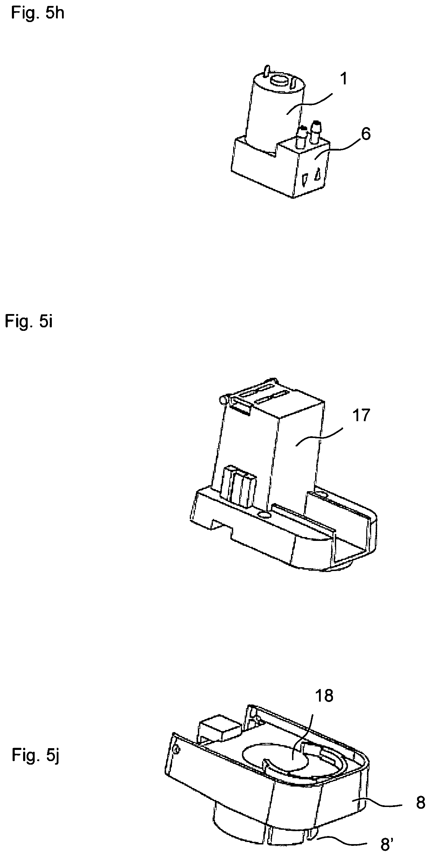

Die in Fig. 1a, b und c gezeigten Fluid-Applikationsvorrichtungen arbeiten hingegen mit Vorratsbehältern 30', aus denen das Fluid durch die Nutzung einer Pumpe 1, siehe Fig. 4 und Fig. 5h gefördert wird. Vorliegend handelt es sich dabei um eine Membranpumpe 1, die mit einem Einwegventil 6 gekoppelt ist und über einen Einlass verfügt, der mittels eines Schlauchstücks das Fluid aus dem Vorratsgefäß 30', siehe Fig. 1b, fördert. Weiter ist die Pumpe auslassseitig über ihren Stutzen mittels eines Schlauches 5" fluidisch mit der Düse 5 beziehungsweise über den Verbindungsstutzen 5', an dem die Düse 5 angeordnet ist, siehe Explosionsansicht Fig. 4, verbunden. Die Ansicht in Fig. 4 zeigt eine Anordnungsvariante für das Batteriemagazin 2 in vertikaler Ausrichtung, nicht vorgesehen zur unmittelbaren Anordnung unter der Abdeckung 16 der Applikationskopfes. Die Fluidverbindung an der Düse 5 über den Stutzen 5' ist auch aus Fig. 2c und Fig. 2f ersichtlich.

Wie zu sehen ist, weist in diesen Ausführungsformen der Applikationskopf 9' an der Oberseite einen als Extremität ausgebildeten Applikationsarm 15 auf, an dessen Unterseite die Düse 5 zur Ausgabe des Fluids angeordnet ist. Wie Fig. 1a zeigt, kann ein Sensor 4 an der vertikalen Wandung des Applikationskopfes 9' vorgesehen sein, alternativ könnte der Sensor 4 jedoch auch unmittelbar hinter der Düse 5 oder auch vor der Düse 5 bei Verwendung eines anderen Designs angeordnet sein. Es ist auch möglich, mehr als einen Sensor 4 zum Einsatz zu bringen, wenn entsprechend weitere Aufgaben außer dem Auslösen der Pumpe mit dem Sensor 4 vorgesehen sind. Weitere Aufgaben könnten etwa die Bestimmung der zu desinfizierenden oder mit Fluid zu versorgenden Körperteilgröße oder deren Form sein. In diesem Fall verfügt der Applikationskopf 9' quasi unmittelbar durch mehrere optische Sensoren, die Reflexionsmessungen erlauben, über Vorrichtungen zur Körperteilerkennung.

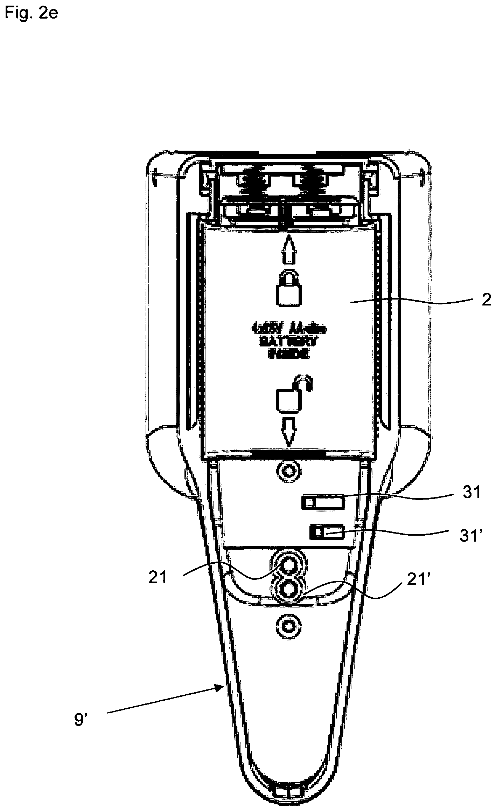

Wie weiter zu sehen ist, erstreckt sich von dem Applikationskopf 9' über ein Adapterstück 8 ein Mantelstück 9 nach unten, das vorzugsweise und um eine geeignete Optik zu schaffen, das Vorratsgefäß zumindest teilweise umgibt und sich vorteilhaft, wie in den Ausführungsformen der Fig. 1a, b und c gezeigt, bis zum unteren Boden des Vorratsgefäßes 30' erstreckt. Die gezeigten Ausführungsformen erlauben das Einführen des Vorratsgefäßes 30' von vorne unten in die Applikationsvorrichtung, so dass das Mantelstück 9 das Vorratsgefäß 30' jeweils umgreift. Von unten erstreckt sich dabei das Vorratsgefäß 30' bis hin zu einem Adapterstück 8, das in Fig. 4 beziehungsweise Fig. 5j deutlich zu sehen ist. Dieses Adapterstück 8 hat einen vom Rand nach innen versetzten Flansch 8', der mit einer Öffnung 18 des Vorratsgefäßes in Eingriff gebracht wird. Weiter bildet das Adapterstück 8 einen Boden, auf den die Pumpe 1, siehe Fig. 5h, sowie ein Batteriemagazingehäuse 17 (Fig. 5i) zur Aufnahme eines Batteriemagazins 2, siehe Fig. 5f, gehalten werden kann, wenn es nicht unmittelbar unter der Abdeckung 16 liegend untergebracht wird (Fig. 2e). Dieser Boden hat eine Durchlassöffnung 18, durch die ein Förderschlauch gesteckt werden kann.

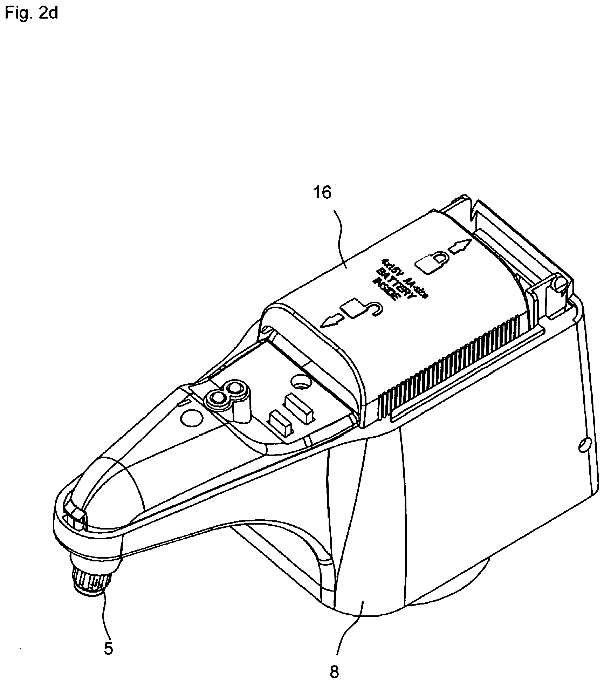

Wie Fig. 2c (geöffneter Applikationskopf 9') zeigt, kann das Batteriemagazin 2 auch zuoberst in dem Applikationskopf 9' angeordnet sein, wobei die Batterien liegend angeordnet sind. Diese Anordnung des Batteriemagazins 2 zeigt auch Fig. 2d, die ferner die LEDs 21,21' zur Anzeige der Betriebsbereitschaft der Vorrichtung und auch die Betätigungsdruckknöpfe 31, 31' zum An- und Ausschalten der Vorrichtung zeigt, die ebenfalls mit dem Steuer- und/oder Regelkreis der Vorrichtung, in geeigneter Weise über die Platine 3, operativ verbunden sind.

Auch Fig. 2e zeigt einen Einblick in den Applikationskopf 9' mit den LEDs 21,21', dem Batteriemagazin 2 und den elektronischen Anschlüssen der Batterien sowie die Betätigungsknöpfe 31,31'.

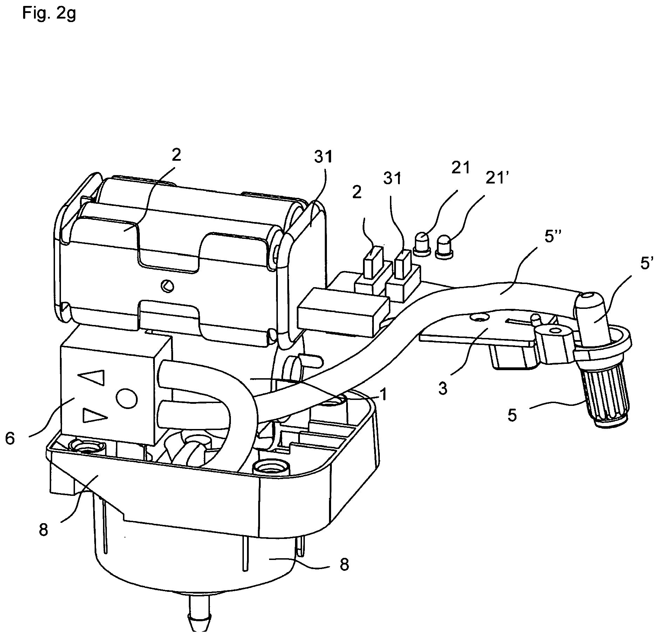

Die perspektivische Seitenansicht des Kopfs der Fluid-Applikationsvorrichtung 9' aus Fig. 2f stellt die Verbindung von der Düse 5 über den Stutzen 5', der mit dem Schlauch 5" verbunden ist, bis zu dem Einwegventil 6 und der Pumpe 1 und schließlich zu dem Stopfen 7 dar, der in das Vorratsgefäß mündet, gegebenenfalls über das Adapterstück 8, wie es ergänzend in Fig. 2g gezeigt ist. In der gezeigten Ausführung sind ferner die Betätigungsdruckknöpfe 31, 31' an der Platine 3 und die LEDs 21,21', die zeigen, ob die Vorrichtung betriebsbereit ist oder nicht, gezeigt.

Es wird vorgeschlagen, zum sicheren und dichten Halten des Förderschlauches einen Stopfen 7, der entsprechend durchbohrt ist, wie in Fig. 5g gezeigt, in die Durchtrittsöffnung 18 einzusetzen. Der Förderschlauch sitzt damit sicher in der Bohrung des Stopfens 7 und dieser in der Öffnung 18 des Adapterstückes 8.

Fig. 5f zeigt im Übrigen mit dem Bezugszeichen 4 versehen als separates Element den Sensor 4. Um die Bauteile aus dem Applikationskopf 9' auf geeignete Weise herausnehmen zu können, kann der Applikationskopf 9' mit einer Abdeckung 16 versehen werden. Dies kann auf verschiedenste Weise, entweder durch Eingriff mittels gezeigter Flansche oder auch durch ein Scharnierelement, oder auf andere Weise aufgesetzt und mit dem weiteren Gehäuse des Applikationskopfes 9' verbunden sein. So kann durch einfaches Öffnen des Applikationskopfes 9' etwa das Batteriemagazin 2 durch Entnahme aus dem Gehäuse 17 ausgetauscht werden.

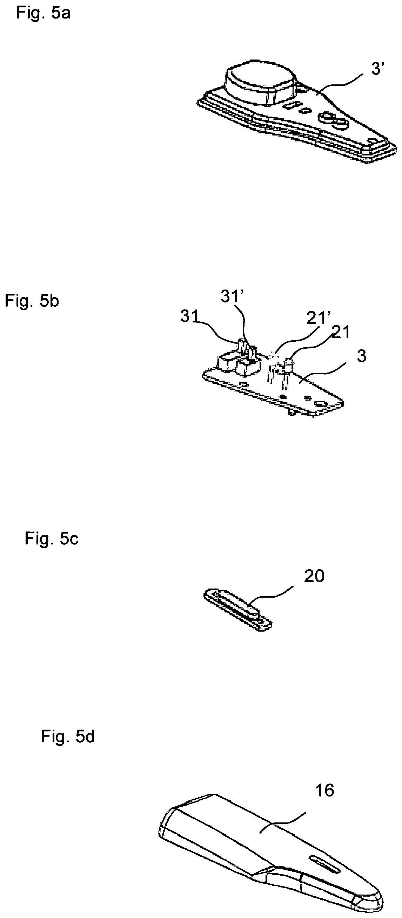

Fig. 5b zeigt (siehe auch Fig. 4) eine Platine 3, auf der eine Steuer- und Regelungseinheit angeordnet ist, die in operativer Verbindung mit dem Sensor 4 steht. Der Sensor 4 kann, figurativ nicht gezeigt, an der Unterseite der Platine 3 angeordnet sein.

Wird nunmehr der Sensor aktiviert, der ein optischer oder optoelektronischer Sensor sein kann und der somit bei Änderung der Lichtverhältnisse, etwa ausgelöst durch das Einschieben einer Hand unter den Sensor, detektiert, so beginnt über die operative Verbindung mittels der Steuerung und Regelung die Pumpe 1 zu arbeiten und bewirkt durch entsprechendes Öffnen des Ventils 6 das Fördern von Fluid aus dem Vorratsgefäß 30' und Ausgeben des Fluids über die Düse 5.

Die Steuerungs- und Regelungsvorrichtung erlaubt entweder das Voreinstellen fester Ausgabevolumina, ausgedrückt in ml, um sicherzustellen, dass ein Betätiger eine hinreichende Menge an Desinfektionsmittel oder Pflegefluid abnimmt, oder sie kann eine Steuerungs- und Regelungsvorrichtung sein, die in Abhängigkeit einer Handgröße oder einer Fluidbeschaffenheit in Verbindung mit der Handgröße oder Hautbeschaffenheit, die durch den optoelektronischen Sensor gemessen werden kann, geregelt wird. Daher ist es auch möglich, eine Mehrzahl an Sensoren 4 vorzusehen, um etwa auf einfache Weise die Umrisse einer Hand optisch zu bestimmen. So wird vorteilhaft mit der Erfindung sichergestellt, dass eine zuverlässige Ausgabe an Desinfektionsmittel in Bezug auf die erforderliche handgrößenabhängige Versorgung geleistet wird. Die Platine 3 kann mit einer Abdeckung 3', siehe Fig. 5a, in dem Applikationskopf 9' geschützt werden. Im Detail zeigt Fig. 5b die Platine mit den Betätigungsknöpfen 31,31' und den LEDs 21,21', die den Betriebszustand anzeigen. Die Platinenabdeckung 3' hat für diese Elemente entsprechende Aufnahmen und Durchlässe. Fig. 5c zeigt das Sichtfenster 20, durch das von außen die LEDs 21,21' gesehen werden können. Die Abdeckung 16 des Applikationskopfes 9' hat eine Ausnehmung für das Sichtfenster 20, siehe Fig. 5d .

Um weiter sicherzustellen, dass der Vorratsbehälter rechtzeitig ausgetauscht wird, kann bei allen Ausführungsformen der Applikationsvorrichtung 90, 100 beispielsweise bei einer fest voreingestellten Ausgabemenge ein Counter im Applikationskopf 9' oder als separates Handgerät vorgesehen sein, das mit einem Signalgeber im Applikationskopf kommuniziert. Der Counter zählt die ausgegebenen Fluiddosen und kann somit das Entleeren des Vorratsgefäßes 30,30' mit verfolgen. Durch eine entsprechende kommunikative Verbindung des Counters mit einer Empfangs- oder Auslesevorrichtung, die das gezählte beziehungsweise das ermittelte Ergebnis anzeigt oder auslesbar macht, kann etwa eine Person, die für das Nachfüllen der Vorratsbehältnisse zuständig ist, feststellen, wie viele Dosen oder welche Menge ausgegeben wurden. Solche Counter können über Radiofrequenz kommunizieren. Ferner kann die erfindungsgemäße Vorrichtung programmiert werden, wenn ein Chip auf der Platine dies zulässt, etwa um Ausgabedosen an Fluid einzustellen oder einen Warnton bei Entleerung zu definieren. Eine solche Programmierung kann über ein Handgerät erfolgen, das auch Bestandteil der Counter-Vorrichtung ist.

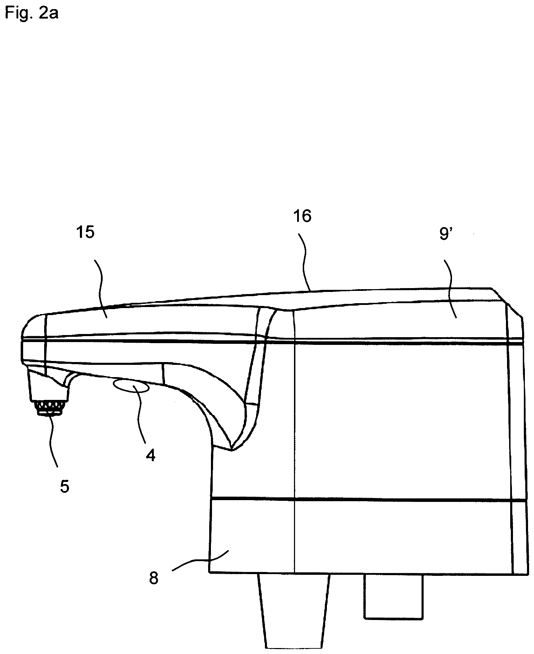

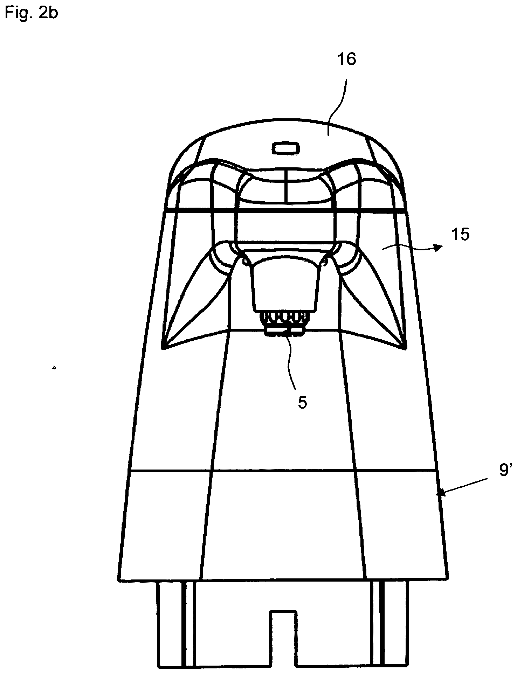

Selbstverständlich können die verschiedenen Applikationsvorrichtungen 90, 100 mit entsprechenden Codes versehen sein, so dass für eine Person, die für das Nachfüllen zuständig ist, eindeutig klar ist, welche der Applikationsvorrichtungen 90,100 der Nachfüllung bedarf. Schließlich zeigen Fig. 2a und 2b den Applikationskopf 9', Fig. 2b zeigt einen vom Rand zurückgesetzten Flansch 8' des Adapterstückes 8, das an dem Applikationskopf angeordnet ist, zum Einsatz in die Öffnung eines Vorratsgefäßes 30'. Weiter zeigt Fig. 2b ein an der Abdeckung 16 des Applikationskopfes 9' vorliegendes Sichtfenster 20, das es ermöglicht, Einblick in den Applikationskopf 9' zu nehmen und festzustellen, ob die grüne LED 21 (siehe Fig. 21) leuchtet, die anzeigt, dass das Gerät betriebsbereit ist, oder ob die rote LED 21' leuchtet, die anzeigt, dass das Gerät nicht betriebsbereit ist. Die LEDs 21, 21' können mit dem Steuer- und Regelkreis verschaltet sein. Selbstverständlich sind andere Farben für die LEDs 21, 21' auswählbar oder es kann ein Blink- oder ein anderer Anzeigemodus eingerichtet werden.

Falls der Counter optisch auslesbare Signale ausgibt, kann er über das Sichtfenster 20 mittels des entsprechenden Auslesegeräts ausgelesen werden.

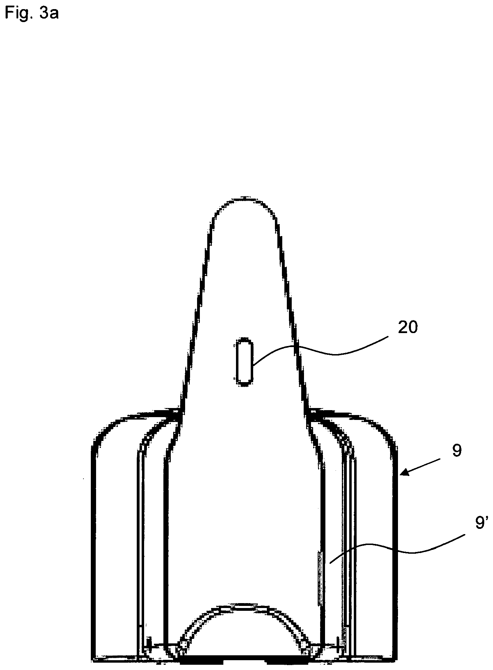

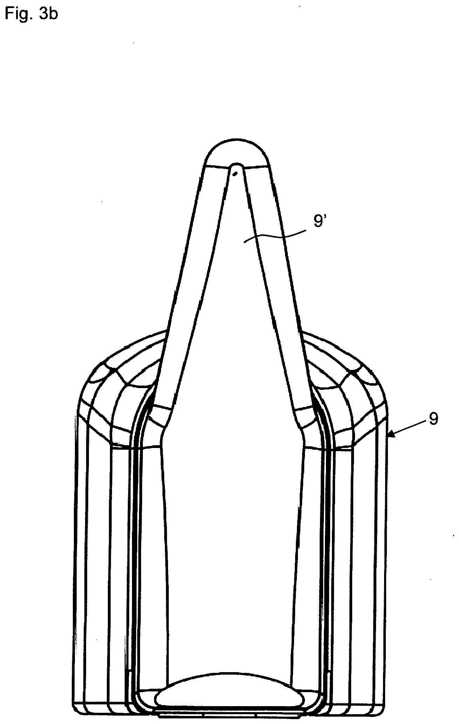

In Fig. 3a ist ebenfalls eine erfindungsgemäße Applikationsvorrichtung 100 mit dem Sichtfenster 20 gezeigt, bei der sich zur Aufnahme großer Vorratsgefäße das Mantelstück 9 nach unten hin weitet, wohingegen die Draufsicht auf eine derartige Vorrichtung in Fig. 3b von oben eine Ausführungsform mit einem etwas schmaleren Mantelelement 9 zeigt. Das weitere Mantelstück ist auch in Fig. 1c im Vergleich zu Fig. 1b verdeutlicht, bei der der Umfang von Mantelelement 9 und Applikationskopf 9' in etwa gleich ist.

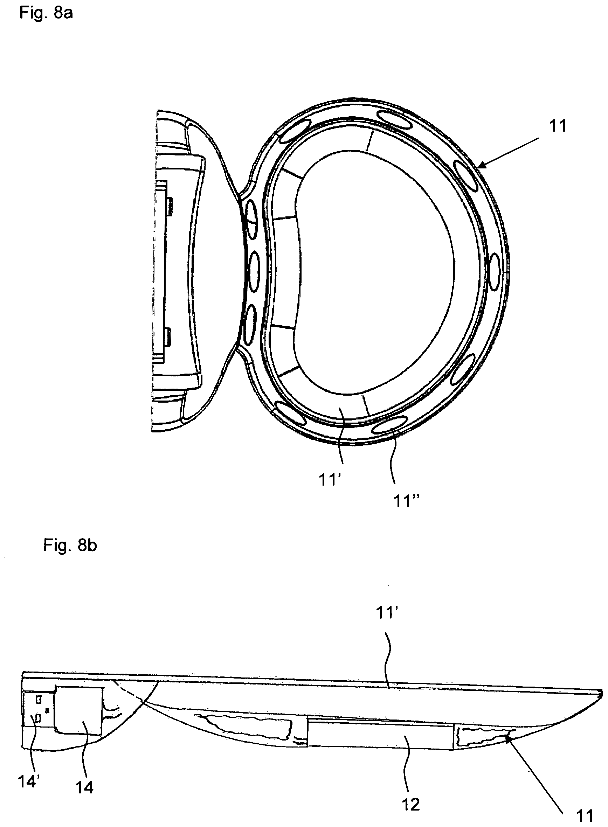

Weiter zeigt Fig. 8a in der Draufsicht eine Auffangschale 11, die sich unmittelbar an den unteren Rand des Mantelstücks anschließt und die dazu dient, überschüssig ausgegebenes Fluid aufzunehmen. Wie Fig. 8b zeigt, ist die Auffangschale 11 als doppelbödiges Element ausgeführt, an ihrer Oberseite des oberen Bodens 1" weist sie Durchtrittsöffnungen 11' auf, durch die das Fluid nach unten abfließen kann.

Grundsätzlich können zum Absorbieren des Fluids auf dem unteren Boden, also zwischen den beiden das doppelbödige Element bildenden Böden, Absorptionsmittel wie Pads, aber auch partikuläres Material oder Saugvlies vorgesehen sein.

Weiter zeigt die Seitenansicht der Fig. 8a ein elektronisches Anschlussstück links am Rand, das an der Verbindung der Auffangschale 11 zu dem Mantelelement 9 elektronische Verbindungsstücke (E-Kontaktgeber und -Stecker 14,14') hat.

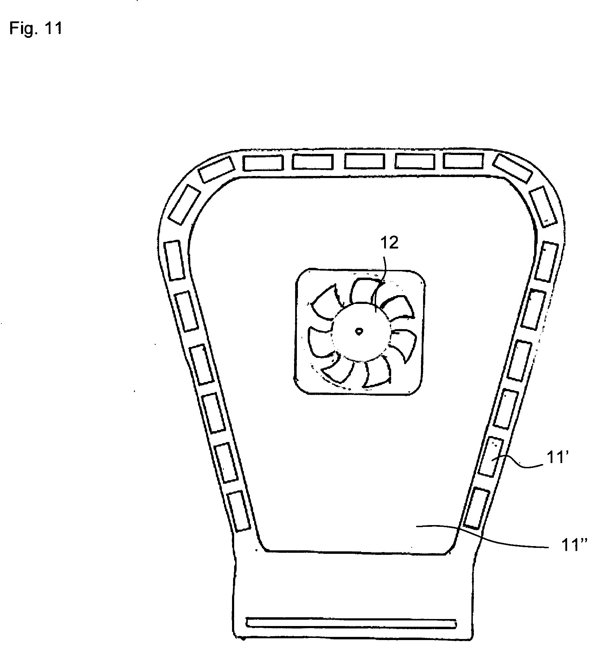

Ferner ist dort ein Saugrotor 12 gezeigt, der durch entsprechende elektronische Kopplung des Antriebs mit dem Sensor 4 unmittelbar in Gang gesetzt werden kann, wenn der Sensor die Fluidausgabe auslöst. Sobald die Absaugvorrichtung in Gang gesetzt wird, wird das überschüssige Fluid durch die Auffangschale 11 sicher aufgenommen und bei entsprechender Ausgestaltung dort sogar zu einem Auffangreservoir gezogen. Dieses kann eben mit dem entsprechenden Absorptionsmittel versehen sein. Während Fig. 8a und 8b eine annähernd ovale Auffangschale 11 zeigen, ist eine andere Designgestaltung der Auffangschale 11 in Fig. 11 abgebildet, auch dort ist ein Absaugrotor 12 vorgesehen. Ein Oberboden 11' der hier doppelbödig ausgeführten Auffangschale 11 hat am Rand die Durchtrittsöffnungen 11", um Fluid abfließen zu lassen; er kann aber auch als Sieb oder Netzbauteil ausgeführt sein.

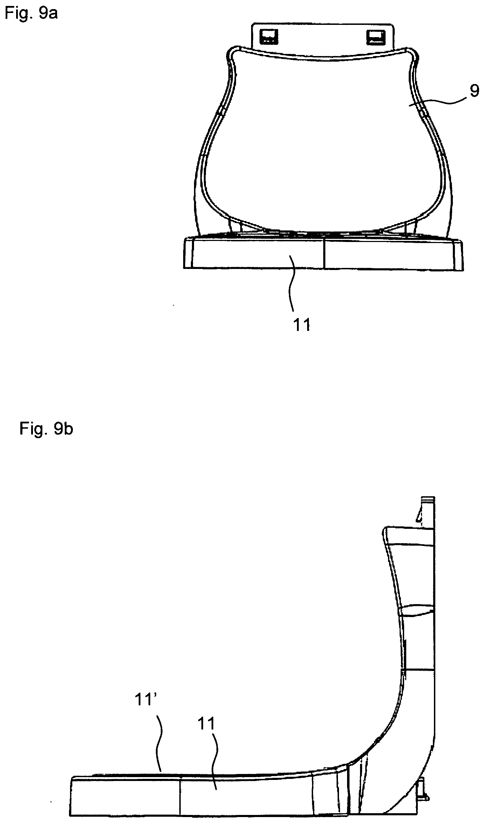

Fig. 9a zeigt den Übergang der Auffangschale 11 in das Mantelelement 9' in eleganter geschwungener Ausführungsform, die auch in Fig. 9b von der Seite zu sehen ist, die Auffangschale 11 geht dabei harmonisch unmittelbar in das Mantelstück 9' über. Eine einbödige Gestaltung der Auffangschale 11 ist möglich, wenn ein Halteelement ein unter der Auffangschale 11 vorgesehenes Fluidabsorptionsmittel hält; in Fig. 9b ist jedoch ein durchlässiger Oberboden 11' gezeigt.

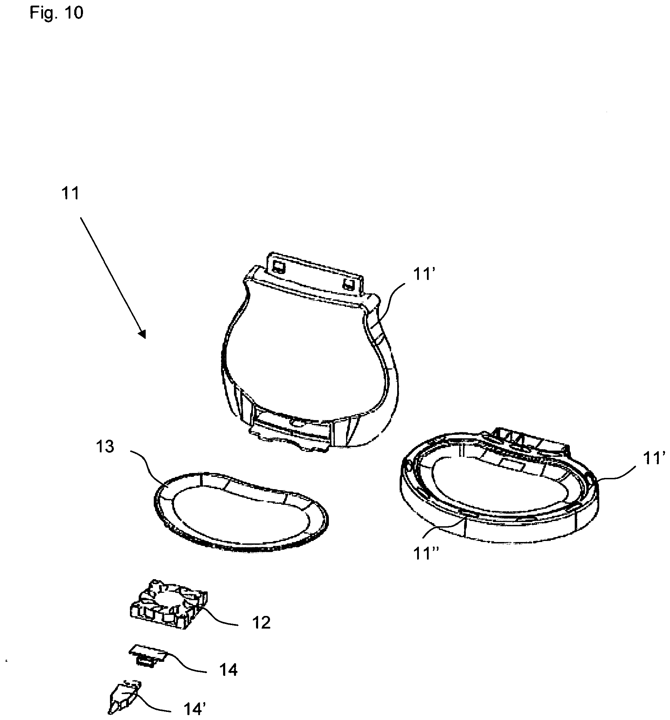

Bei der Explosionsansicht in Fig. 10 ist eine Ausgestaltung der Auffangschale 11 nochmals verdeutlicht: Die Oberböden der Auffangschale 11 sind in stehender und liegender Position in der Mitte beziehungsweise rechts zu sehen, während Bezugszeichen 13 als Haltemittel für ein Pad eine unter den Oberboden 11' klemmbare Schale 13' zeigt. Bezugszeichen 12 bezeichnet den Rotor und die Bezugszeichen 14, 14' elektronische Anschlusselemente beziehungsweise den Kontaktgeber und den E-Kontaktstecker 14'.

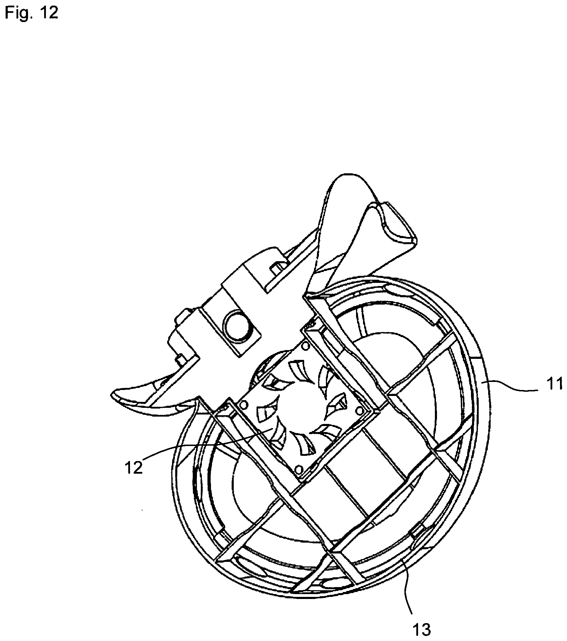

Fig. 12 zeigt eine Ansicht einer solchen mit Saugrotor 12 ausgestatteten Auffangschale von unten.

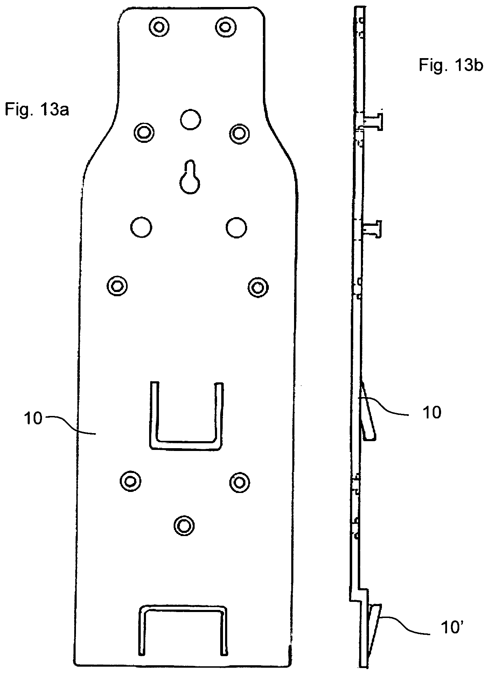

Fig. 13a und 13b zeigen zwei Ansichten eines geeigneten Wandaufhängungselements bzw. einer Platte, die insbesondere dazu geeignet ist, eine Fluid-Applikationsvorrichtung 90, 100 an einer Wand zu haltern. Die Wandhalterung 10 kann, wenn erforderlich, über Durchführöffnungen für elektronische Leitungen verfügen. Eine Klemme 10' dient dazu, eine Auffangschale 111, die nicht an einem Mantelstück 9 angebracht ist, unter dem Applikationskopf 9' oder auch der Vorrichtung mit Mantelstück 9 zu halten.

Mit der Schraubklemme 19 ausgestattet, kann die Wandhalterung 10 aber auch zur Anbringung der Fluid-Applikationsvorrichtung 90, 100 an einem Bett, Tisch, Regal oder ähnlichem dienen und so in geeigneter Reichweite für einen avisierten Nutzer angebracht sein.

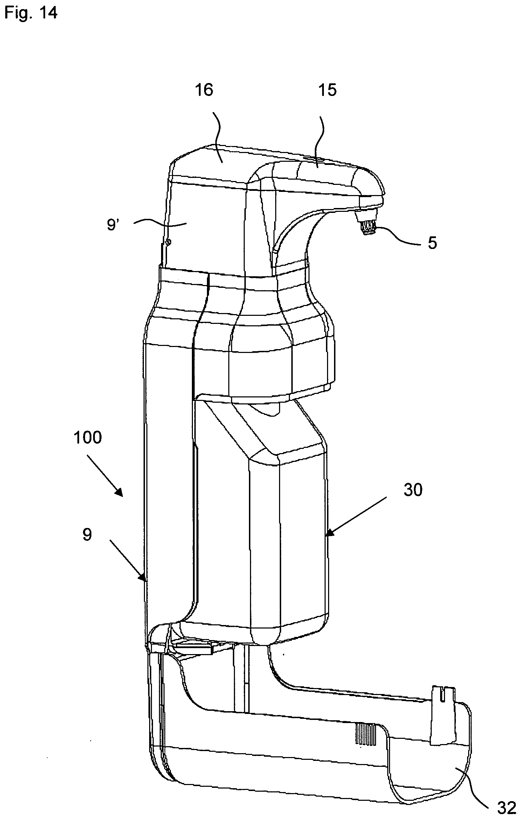

Fig. 14 und Fig. 1d zeigen schließlich, dass das Mantelstück 9 durchaus auch so beschaffen sein kann, dass das Vorratsgefäß 30' abgedeckt sein kann. Hierzu hat das Mantelstück 9 einen vorderen gelenkig aufklappbaren Abschnitt 32. Das Gelenk ist hier ein Scharnier, das am unteren Rand des Mantelstücks 9 angebracht ist und das es erlaubt, die Applikationsvorrichtung 100 zum einfachen Vorratsgefäßaustausch aufzuklappen. Die geschlossene Variante zeigt Fig. 1d, während Fig. 14 die aufgeklappte Variante zeigt.

Alternativ kann der vordere Abschnitt des Mantelstücks 9 auch über eine Schienenführung aufschiebbar oder aufsteckbar sein.

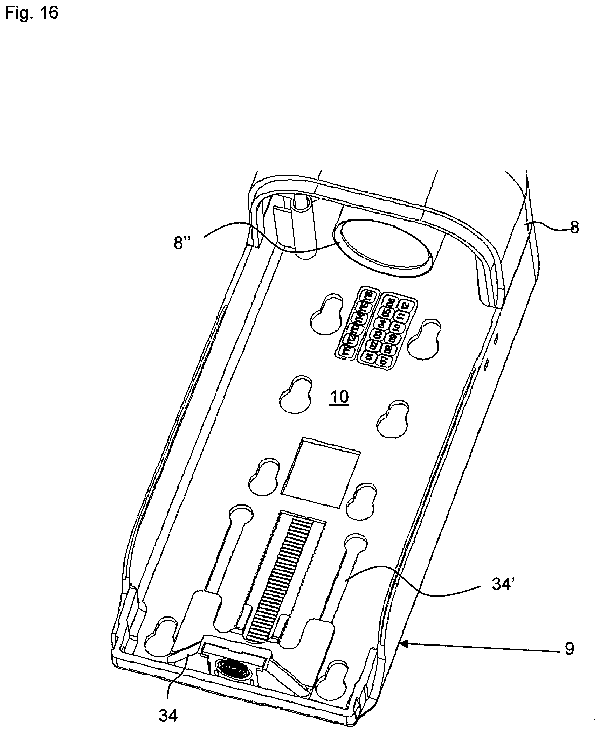

Das Mantelstück 9 kann ferner so ausgebildet sein, dass es an seiner Innenseite zur flexiblen Nutzung der Vorrichtung einen Lift aufweist, der das Anheben kleiner Vorratsgefäße 30' bis hin zum Adapter 8 erlaubt. Eine solche Ausgestaltung, hier als einstückig ausgebildete Wandhalterung 10 mit Mantelstück 9, ist in Fig. 16 gezeigt: Die Liftvorrichtung umfasst eine Aufstandsfläche 34 für das Vorratsgefäß 30' und ein Schienenelement 34', so dass die Aufstandsfläche einen vertikal verschiebbaren Boden an dem Mantelstück 9 bildet. Damit können kleine Flaschen einfach auf die Aufstandsfläche 34 gestellt und zu dem Adapterstück 8 hingefahren werden.

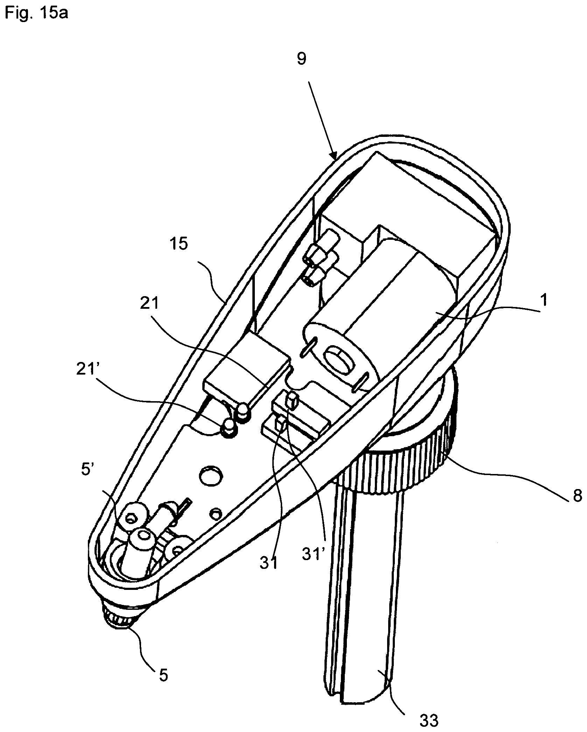

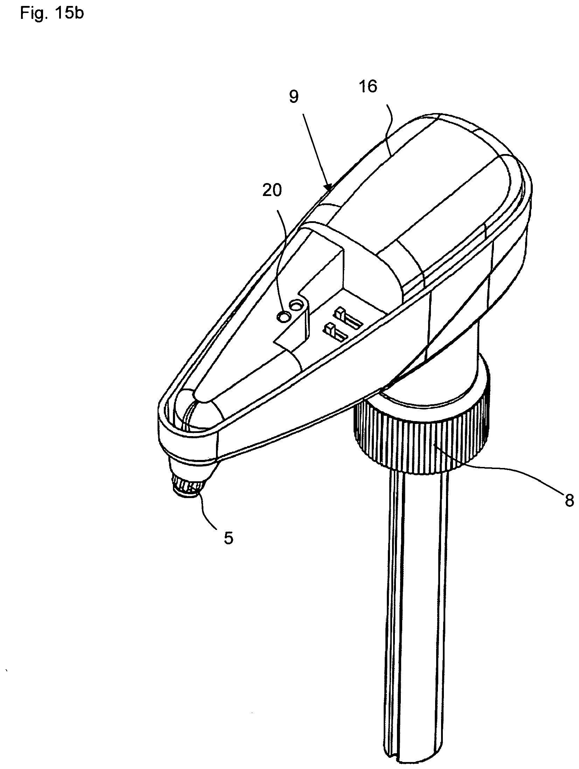

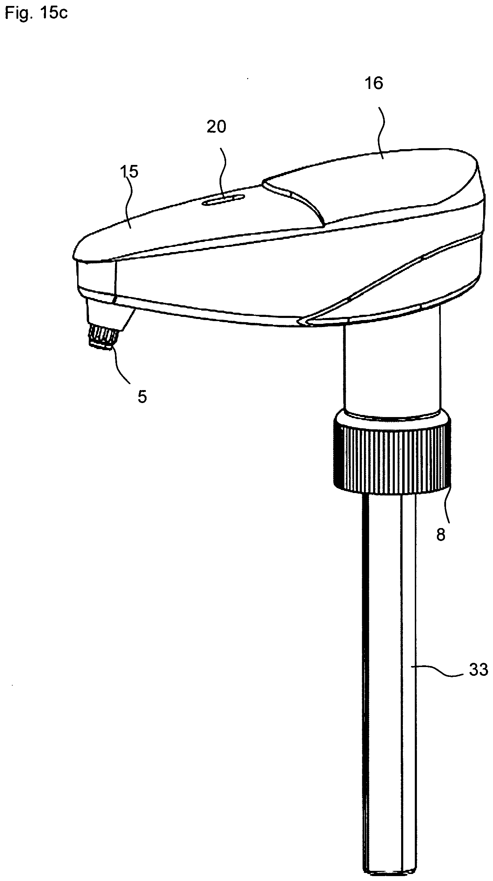

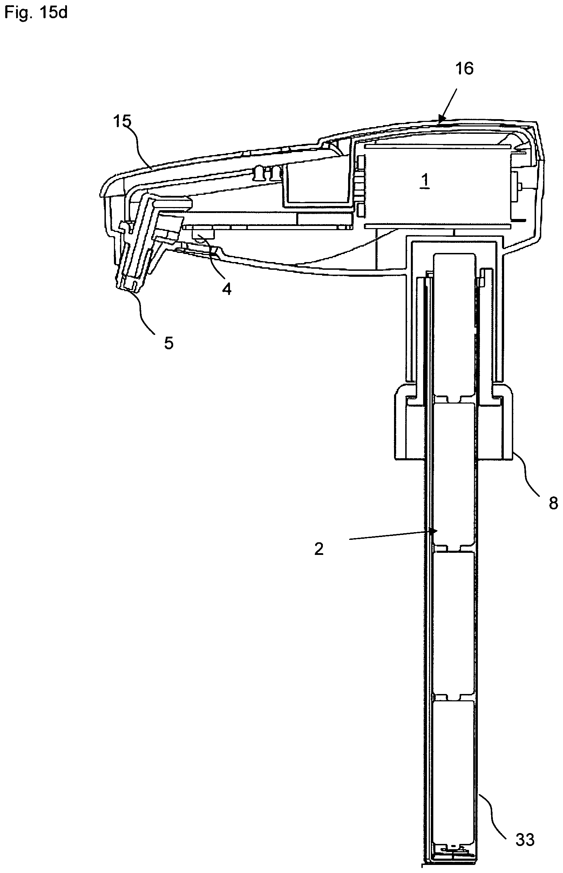

Eine Ausgestaltung der Fluid-Applikationsvorrichtung mit einem Adapterstück 8 als Überwurfmutter ist in Fig. 15 b bis d gezeigt. Die Überwurfmutter ist über ein Aufsteckstück mit einer Umfangsnut auf einen unteren Stutzen an dem Applikationskopf 9' aufgesteckt und so mit diesem verbunden. Wie insbesondere aus Fig. 15d zu sehen ist, ist das Fördermittel dort ein Koaxial-Förderrohr 33, dessen Innenrohr das Batteriemagazin 2 bildet, das zur Energieversorgung mit der Pumpe 1 und dem Sensor 4 verbunden ist. Die Pumpe 1 ist liegend im Kopf 9' angeordnet, der einen formschönen schmalen Applikationsarm 15 hat.

Für sicheren Stand kann eine Vorratsflasche mit Desinfektionsmittel oder einem anderen Fluid, die mit dieser Applikationsvorrichtung ausgestattet ist, in eine Halterung zur Verbesserung der Standsicherheit eingesetzt werden.

### Bezugszeichenliste

- 1: Motor/Pumpe
- 2: Batteriemagazin
- 3: Platine
- 3': Platinenabdeckung
- 4: Sensor
- 5: Düse
- 5': Verbindungsstutzen
- 5": Schlauch
- 6: Einwegventil
- 7: Silikonstopfen mit Doppellochführung
- 8: Adapter für Applikationsvorrichtung
- 8': Flansch
- 9: Gehäuse, Mantelstück der Applikationsvorrichtung
- 9': Applikationskopf
- 10: Wandhalterung
- 10': Klemme für Auffangschale
- 11: Auffangschale
- 11': Oberboden Auffangschale
- 11": Durchtrittsöffnungen Oberboden
- 12: Saugrotor
- 13: Klemmschale
- 14: E-Kontakt-Geber
- 14': E-Kontakt-Stecker
- 15: Applikationsarm
- 16: Abdeckung des Applikationskopfes
- 17: Batteriemagazingehäuse
- 18: Adapteröffnung
- 19: Schraubklemme
- 20: Sichtfenster
- 21: Grüne LED
- 21': Rote LED
- 22: Aufsatz mit Minikopf und Klemmhalterung für Aerosoldose
- 22': Abstützflansch
- 24: Klemmhalterung
- 25: Klemmfeder
- 26: Stellschraube
- 27: Umgriffelement
- 28: Rahmenteil
- 30: Vorratsgefäß, Flasche, Dose
- 31: Betätigungsdruckknopf
- 31': Betätigungsdruckknopf
- 32: Angelenkter vorderer Mantelstückabschnitt
- 33: Förderrohr, Koax-Förderrohr
- 34: Lift-Auflage
- 34': Schienenelement für Lift
- 90: Applikationsvorrichtung
- 100: Applikationsvorrichtung

## Patentansprüche

1. Fluid-Applikationsvorrichtung (90) mit einer Fluidausgabe, die einen Vorratsbehälter (30), ein Gehäuse (9), ein Adapterstück zum Halten des Vorratsbehälters (30) und einen Applikationskopf (9') umfasst, der oberhalb des Vorratsbehälters (30') angeordnet ist und eine Düse (5) zum Ausgeben von Fluid aufweist, wobei
- die Düse mit einem Vorratsbehälter (30) fluidisch verbunden ist,
- der Vorratsbehälter (30) ein unter Überdruck stehendes Gefäß (8') ist, das einen Auslass aufweist, der durch ein Ventil öffen- und schließbar ist und
- das Gehäuse (9) und der Applikationskopf (9') separate Bauteile sind
**dadurch gekennzeichnet, dass** der Applikationskopf (9') an seiner Oberseite einen Applikationsarm (15) aufweist, an dem nach unten weisend die Düse (5) angeordnet ist, wobei das Adapterstück zum Halten des Vorratsbehälters (30) an dem Applikationskopf (9') angeordnet ist und eine Klemmhalterung aufweist, die zwei mittels jeweils einer Klemmfeder (25) vorgespannte Umgriffelemente (27), ein Rahmenteil (28) und eine Betätigungsvorrichtung zum Vorspannen und Lösen der Klemmfedern (25) umfasst, und die Fluid-Applikationsvorrichtung eine automatische Fluidausgabe aufweist, wobei in dem Applikationskopf (9') zumindest ein Sensor (4) und eine Steuereinheit zum sensorgesteuerten Öffnen und Schließen des Ventils vorliegen, das über ein Verbindungsstück mit der Düse (5) zur Ausgabe des Fluids verbunden ist.

2. Fluid-Applikationsvorrichtung (100) nach Anspruch 1,
**dadurch gekennzeichnet, dass**
die zwei mittels jeweils einer Klemmfeder (25) vorgespannten Umgriffelemente (27) in einem Rahmenteil (28) radial verschiebbar angeordnet sind, wobei die Klemmfedern (25) außenseitig gegen
- einen Aufsatz (22), der an dem Applikationskopf (9') anordenbar ist, oder
- gegen eine Innenwandung des Applikationskopfs (9') abgestützt angeordnet sind.

3. Fluid-Applikationsvorrichtung (100) nach Anspruch 1 oder 2 **dadurch gekennzeichnet, dass** die Betätigungsvorrichtung eine Stellschraube (26) ist.

4. Fluid-Applikationsvorrichtung (100) mit automatischer Fluidausgabe, die einen Vorratsbehälter (30') und einen Applikationskopf (9') umfasst, der oberhalb des Vorratsbehälters (30') angeordnet ist und zumindest einen Sensor (4) und eine Düse (5) zum Ausgeben von Fluid aufweist, wobei der Sensor (4) mit einer mit der Düse (5) verbundenen Pumpe (1), die an dem Vorratsbehälter (30') angeschlossen ist, operativ verbunden ist, wobei der Applikationskopf (9') einen Applikationsarm (15) aufweist, an dem nach unten weisend die Düse (5) angeordnet ist, und wobei in dem Applikationskopf (9') die Pumpe (1), die durch den Sensor (4) steuerbar und motorbetrieben ist, mit einem Ein- und einem Ausgang angeordnet ist und über ein Ventil (6) ausgangsseitig mittels einer Schlauch- oder Rohrverbindung (5") mit einem Verbindungsstutzen (5') der Düse (5) zur Ausgabe des Fluids verbunden ist, **dadurch gekennzeichnet, dass** ein Gehäuse (9) und ein Adapterstück (8) zum Halten des Vorratsbehälters vorgesehen sind, wobei das Gehäuse (9) und der Applikationskopf (9') separate Bauteile sind, wobei das Adapterstück (8) an dem Applikationskopf (9') angeordnet ist und ein äußerer Rand des Adapterstücks (8') mit dem Gehäuse (9), das zumindest einen Abschnitt einer rückwärtigen Seite des Vorratsbehälters (30,30') umgibt, verbunden ist, wobei der Applikationsarm (15) an der Oberseite des Applikationskopfes (9') angeordnet ist, und wobei die Pumpe (1) über dem Adapterstück (8) angeordnet ist, mittels dessen der Applikationskopf (9') mit dem Vorratsbehälter (30') fluiddicht verbindbar ist.

5. Fluid-Applikationsvorrichtung (100) nach Anspruch 4,
**dadurch gekennzeichnet, dass** das Adapterstück (8) an dem Applikationskopf (9') dauerhaft oder lösbar angeordnet ist, wobei eine Unterseite des Adapterstücks (8) mit einer Öffnung des Vorratsbehälters (30') in Eingriff bringbar ist, und wobei sich eine Fördervorrichtung, insbesondere ein Schlauch oder ein Rohr (33) von der Eingangsseite der Pumpe (1) in den Vorratsbehälter (30') erstreckt.

6. Fluid-Applikationsvorrichtung (100) nach Anspruch 4 oder 5,
**dadurch gekennzeichnet, dass** das Adapterstück (8) eine Oberseite aufweist, die formschlüssig mit dem Applikationskopf (9') verbindbar ist und dessen Unterseite einen Flansch aufweist, insbesondere einen Flansch (8'), der von einem Rand des Adapterstücks nach innen versetzt ist und die mit der Öffnung des Vorratsbehälters (30') in Eingriff bringbar ist, und wobei die Fördervorrichtung ein Förderschlauch ist, der sich von der Eingangsseite der Pumpe (1) durch eine in dem Adapterstück (8) vorgesehene Öffnung (18) in den Vorratsbehälter (30') erstreckt.

7. Fluid-Applikationsvorrichtung (90, 100) nach einem der vorhergehenden Ansprüche, **dadurch gekennzeichnet, dass** der Sensor (4) oder die Sensoren (4) insbesondere optische oder opto-elektronische Sensor(en) (4) ist/sind, wobei zumindest ein Sensor (4) an dem Applikationsarm (15) nach unten weisend oder an dem Applikationskopf (9') in Richtung der Düse (5) weisend angeordnet ist.

8. Fluid-Applikationsvorrichtung (100) nach zumindest einem der Ansprüche 4 bis 7
**dadurch gekennzeichnet, dass** die operativ durch den Sensor/die Sensoren betreibbare Pumpe eine Membranpumpe ist, die ausgangsseitig mit einem Einwegventil (6) operativ gekoppelt ist, wobei die Membranpumpe mit einer Energieversorgung, insbesondere einem als Gesamtheit austauschbaren Batteriemagazin (2) gekoppelt ist.

9. Fluid-Applikationsvorrichtung (90, 100) nach zumindest einem der Ansprüche 1 bis 8,
**dadurch gekennzeichnet, dass** die Steuerungseinheit zum Steuern des Ventils auch als Regelungseinheit ausgebildet ist, und insbesondere auf einer Platine (3) angeordnet ist, wobei insbesondere ein Ausgabevolumen des Fluids vorbestimmbar ist.

10. Fluid-Applikationsvorrichtung (90, 100) nach einem der vorhergehenden Ansprüche,
**dadurch gekennzeichnet, dass** in dem Applikationskopf (9') ferner
- eine Vorrichtung zur Dichtemessung des Fluids, und/oder
- eine Vorrichtung zur Hand- und oder Hautbeschaffenheitserkennung und/oder
- eine Vorrichtung zur Handgrößenbestimmung angeordnet ist, wobei die Vorrichtung zur Handgrößenbestimmung operativ mit der Steuerungs- und/oder Regelungsvorrichtung gekoppelt ist.

11. Fluid-Applikationsvorrichtung (100) nach zumindest einem der Ansprüche 1 bis 3, oder 5-10, wenn abhängig von 1-3, **dadurch gekennzeichnet, dass**
ein äußerer Rand des Adapterstücks (8') mit einem sich von dem Adapterstück (8) nach unten erstreckenden Mantelstück (9), das zumindest einen Abschnitt einer rückwärtigen Seite des Vorratsgefäßes (30,30') umgibt, verbunden ist.

12. Fluid-Applikationsvorrichtung (90, 100) nach einem der vorhergehenden Ansprüche,
**dadurch gekennzeichnet, dass** das Gehäuse (9) sich zumindest bis unterhalb des Vorratsbehälters(30,30') erstreckt und insbesondere an dem unteren Rand des Gehäuses (9) eine Auffangschale (11) angeordnet ist, die sich entsprechend einer Ausdehnung des Applikationsarms (15) unter diesen erstreckt,
und/oder wobei die Auffangschale (11) einen doppelten Boden aufweist, dessen oberer Boden (11') eine Mehrzahl von Durchtrittsöffnungen (11") für Fluid aufweist,
und wobei bevorzugt eine Absaugvorrichtung, insbesondere ein Saugrotor (12), in der Auffangschale, insbesondere unter dem oberen Boden (11') und in fluidischem Kontakt mit den Durchtrittsöffnungen (11") für Fluid zur Fluidabsaugung angeordnet ist.

13. Fluid-Applikationsvorrichtung (90,100) nach Anspruch 12,
**dadurch gekennzeichnet, dass** in der Auffangschale (11), insbesondere unter dem oberen Boden (11'), der die Mehrzahl von Durchtrittsöffnungen (11") für Fluid aufweist, zumindest ein Absorptionsmittel für Fluid angeordnet ist, wobei das Absorptionsmittel insbesondere ein Saugvlies oder ein Saugpad ist, und/oder dass die Auffangschale (11) lösbar an dem Gehäuse (9) angeordnet ist,
und/oder
dass an einer Anschlussstelle der Auffangschale (11) an das Gehäuse (9) eine Durchführung für eine Energieversorgungsleitung vorgesehen ist.

14. Fluid-Applikationsvorrichtung (100) nach zumindest einem der Ansprüche 4 bis 13, **dadurch gekennzeichnet, dass** das Adapterstück (8) als eine Überwurfmutter ausgebildet und mit dem Applikationskopf (9') verbunden ist und ein Schraubgewinde aufweist, das mit einem an dem Vorratsbehälter (30') vorliegenden Hals-Außengewinde verschraubbar ist,
und wobei die Fördervorrichtung ein doppelwandiges Förderrohr (33), insbesondere ein Koaxial-Förderrohr (33) ist, deren Innenrohr ein Batteriemagazin (2) bildet, das zur Energieversorgung zumindest mit der Pumpe (1), insbesondere mit der Pumpe und einem oder weiteren in dem Applikationskopf (9') vorliegenden Energieverbrauchern elektrisch verbunden ist.

15. Fluid-Applikationsvorrichtung (90,100) nach zumindest einem der vorhergehenden Ansprüche,
**dadurch gekennzeichnet, dass** die Fluid-Applikationsvorrichtung (90, 100)
- als Stand-Anordnung oder
- zur Wandaufhängung, insbesondere zur Wandaufhängung mittels einer an dem Gehäuse (9) angeordneten oder mit diesem einstückig ausgebildete Wandhalterung (10), mit oder ohne elektronische Durchführungen, oder zur Anordnung an einem Möbel oder einem anderen vertikalen Tragarm mittels einer an der Wandhalterung (10) angebrachten Schraubklemme (19) ausgebildet ist.

16. Fluid-Applikationsvorrichtung (90, 100) nach zumindest einem der vorhergehenden Ansprüche,
**dadurch gekennzeichnet, dass** das Gehäuse (9) oder eine mit diesem einstückig ausgebildete Wandhalterung (10) innenseitig eine Liftvorrichtung (34) aufweist, bestehend aus einer Aufstandsfläche (34) die in einem Schienenelement (34') vertikal verschiebbar angeordnet ist.

17. Fluid-Applikationsvorrichtung (90, 100) nach einem der vorhergehenden Ansprüche,
**dadurch gekennzeichnet, dass** das Gehäuse (9) einen vorderen Abschnitt (32) aufweist, der
- gelenkig aufklappbar ist und mit dem es über ein an seinem unteren Rand verbundenes Scharnier verbunden ist, oder der
- über eine Schiene von unten aufschiebbar ist.

## Claims

1. Fluid application device (90) having a fluid dispenser, which device comprises a reservoir (30), a housing (9), an adapter piece for holding the reservoir (30), and an application head (9') which is arranged above the reservoir (30') and has a nozzle (5) for dispensing fluid,
- the nozzle being fluidically connected to a reservoir (30),
- the reservoir (30) being a pressurised vessel (8') which has an outlet which can be opened and closed by means of a valve, and
- the housing (9) and the application head (9') being separate components, **characterised in that** the application head (9') has, on the upper side thereof, an application arm (15) on which the nozzle (5) is arranged so as to point downwards, the adapter piece for holding the reservoir (30) being arranged on the application head (9') and having a clamping bracket which comprises two encompassing elements (27) that are each preloaded by means of a clamping spring (25), a frame part (28) and an actuating device for preloading and releasing the clamping springs (25), and the fluid application device having an automatic fluid dispenser, at least one sensor (4) and a control unit for opening and closing the valve in a sensor-controlled manner being provided in the application head (9'), which valve is connected to the nozzle (5) by means of a connecting piece in order to dispense the fluid.

2. Fluid application device (100) according to claim 1, **characterised in that** the two encompassing elements (27) that are each preloaded by means of a clamping spring (25) are arranged in a frame part (28) so as to be radially slidable, the clamping springs (25) being arranged on the outside so as to be supported against
- an attachment (22) which can be arranged on the application head (9'), or
- an inner wall of the application head (9').

3. Fluid application device (100) according to either claim 1 or claim 2, **characterised in that** the actuating device is an adjusting screw (26).

4. Fluid application device (100) having an automatic fluid dispenser, which device comprises a reservoir (30') and an application head (9') which is arranged above the reservoir (30') and has at least one sensor (4) and a nozzle (5) for dispensing fluid, the sensor (4) being operatively connected to a pump (1) that is connected to the nozzle (5) and is attached to the reservoir (30'), the application head (9') having an application arm (15) on which the nozzle (5) is arranged so as to point downwards, and the pump (1) being arranged in the application head (9') so as to have an inlet and an outlet, which pump is controllable and motor-operated by the sensor (4), and being connected, on the outlet side, to a connecting piece (5') of the nozzle (5) via a valve (6) by means of a hose or tube connection (5") in order to dispense the fluid, **characterised in that** a housing (9) and an adapter piece (8) for holding the reservoir are provided, the housing (9) and the application head (9') being separate components, the adapter piece (8) being arranged on the application head (9') and an outer edge of the adapter piece (8') being connected to the housing (9), which surrounds at least one portion of a rear side of the reservoir (30, 30'), the application arm (15) being arranged on the upper side of the application head (9'), and the pump (1) being arranged above the adapter piece (8), by means of which piece the application head (9') can be connected to the reservoir (30) in a fluid-tight manner.

5. Fluid application device (100) according to claim 4, **characterised in that** the adapter piece (8) is permanently or detachably arranged on the application head (9'), it being possible to bring an underside of the adapter piece (8) into engagement with an opening in the reservoir (30'), and a conveying device, in particular a hose or a tube (33), extending from the inlet side of the pump (1) into the reservoir (30').

6. Fluid application device (100) according to either claim 4 or claim 5, **characterised in that** the adapter piece (8) has an upper side which can be form-fittingly connected to the application head (9') and the underside of which has a flange, in particular a flange (8') which is offset inwards from an edge of the adapter piece and which can be brought into engagement with the opening in the reservoir (30'), and the conveying device being a conveying hose which extends from the inlet side of the pump (1), through an opening (18) that is provided in the adapter piece (8), into the reservoir (30').

7. Fluid application device (90, 100) according to any of the preceding claims, **characterised in that** the sensor (4) or the sensors (4) is/are in particular optical/optoelectronic sensor(s) (4), at least one sensor (4) being arranged on the application arm (15) so as to point downwards or on the application head (9') so as to point towards the nozzle (5).

8. Fluid application device (100) according to at least one of claims 4 to 7, **characterised in that** the pump which can be operated by means of the sensor/sensors is a diaphragm pump which is operatively coupled, on the outlet side, to a one-way valve (6), the diaphragm pump being coupled to a power supply, in particular a battery magazine (2) which is interchangeable as a whole.

9. Fluid application device (90, 100) according to at least one of claims 1 to 8, **characterised in that** the control unit for controlling the valve is also designed as a closed-loop control unit, and in particular is arranged on a circuit board (3), it being possible in particular to predetermine a dispensing volume of the fluid.

10. Fluid application device (90, 100) according to any of the preceding claims, **characterised in that,** in the application head (9'),
- a device for measuring the density of the fluid, and/or
- a device for detecting the condition of a hand and/or skin, and/or
- a device for determining hand size is additionally arranged, the device for determining hand size being operatively coupled to the open-loop and/or closed-loop control device.

11. Fluid application device (100) according to at least one of claims 1 to 3, or 5-10, when dependent on 1-3, **characterised in that** an outer edge of the adapter piece (8') is connected to a jacket piece (9) which extends downwards from the adapter piece (8) and surrounds at least one portion of a rear side of the reservoir vessel (30, 30').

12. Fluid application device (90, 100) according to any of the preceding claims, **characterised in that** the housing (9) extends at least to below the reservoir (30, 30') and a drip tray (11) is arranged in particular on the lower edge of the housing (9) and extends under the application arm (15) so as to correspond to an extension of said arm, and/or the drip tray (11) having a double base, the upper base (11') of which has a plurality of passages (11") for fluid, and preferably a suction device, in particular a suction rotor (12), being arranged in the drip tray, in particular under the upper base (11'), and so as to be in fluid contact with the passages (11") for fluid in order to suction fluid.

13. Fluid application device (90, 100) according to claim 12, **characterised in that** at least one absorbent material for fluid is arranged in the drip tray (11), in particular under the upper base (11') which has the plurality of passages (11") for fluid, the absorbent material being in particular an absorbent fleece or an absorbent pad, and/or **in that** the drip tray (11) is detachably arranged on the housing (9), and/or **in that** a bushing for a power supply line is provided at a connection point of the drip tray (11) on the housing (9).

14. Fluid application device (100) according to at least one of claims 4 to 13, **characterised in that** the adapter piece (8) is designed as a union nut, is connected to the application head (9') and has a screw thread which can be screwed together with a neck external thread that is provided in the reservoir (30'), and the conveying device being a double-walled conveying tube (33), in particular a coaxial conveying tube (33), the inner tube of which forms a battery magazine (2) which is electrically connected at least to the pump (1), in particular to the pump and one or more energy consumers that are provided in the application head (9'), in order to supply power.

15. Fluid application device (90, 100) according to at least one of the preceding claims, **characterised in that** the fluid application device (90, 100) is designed
- as a stand assembly or
- for wall suspension, in particular for wall suspension by means of a wall bracket (10) that is arranged on the housing (9) or is integral therewith, with or without electronic bushings, or for arrangement on a piece of furniture or another vertical support arm, by means of a clamping screw (19) that is affixed to the wall bracket (10).

16. Fluid application device (90, 100) according to at least one of the preceding claims, **characterised in that** the housing (9) or a wall bracket (10) that is integrally formed therewith has a lifting device (34) on the inside, consisting of a contact surface (34) which is arranged in a rail element (34') so as to be vertically slidable.

17. Fluid application device (90, 100) according to any of the preceding claims, **characterised in that** the housing (9) has a front portion (32) which
- can be hingedly folded open and to which said housing is connected by means of a hinge that is connected to the lower edge of said housing, or
- can be slid on from below by means of a rail.

## Revendications

1. Dispositif d'application de fluide (90) avec une distribution de fluide, qui comprend un réservoir de stockage (30), un boîtier (9), une pièce d'adaptation pour maintenir le réservoir de stockage (30) et une tête d'application (9') qui est disposée au-dessus du réservoir de stockage (30') et qui présente une buse (5) pour la distribution de fluide, dans lequel
- la buse est en liaison fluidique avec un réservoir de stockage (30),
- le réservoir de stockage (30) est un récipient sous surpression (8') qui présente une sortie qui peut être ouverte et fermée par une vanne, et
- le boîtier (9) et la tête d'application (9') sont des composants séparés, **caractérisé en ce que** la tête d'application (9') présente sur son côté supérieur un bras d'application (15) sur lequel la buse (5) est disposée en étant dirigée vers le bas, la pièce d'adaptation destinée à maintenir le réservoir de stockage (30) étant disposée sur la tête d'application (9') et présentant un support de serrage qui comprend deux éléments de serrage (27) précontraints chacun au moyen d'un ressort de serrage (25), une partie cadre (28) et un dispositif d'actionnement pour précontraindre et libérer les ressorts de serrage (25), et le dispositif d'application de fluide présente une distribution automatique de fluide, la tête d'application (9') comportant au moins un capteur (4) et une unité de commande pour l'ouverture et la fermeture commandées par capteur de la vanne qui est reliée par une pièce de liaison à la buse (5) pour la distribution du fluide.

2. Dispositif d'application de fluide (100) selon la revendication 1,
**caractérisé en ce que**
les deux éléments de serrage (27) précontraints chacun au moyen d'un ressort de serrage (25) sont disposés de manière à pouvoir coulisser radialement dans une partie cadre (28), les ressorts de serrage (25) étant disposés en prenant appui extérieurement contre
- un élément rapporté (22) qui peut être disposé sur la tête d'application (9') ou
- contre une paroi intérieure de la tête d'application (9').

3. Dispositif d'application de fluide (100) selon la revendication 1 ou 2 **caractérisé en ce que** le dispositif d'actionnement est une vis de réglage (26).

4. Dispositif d'application de fluide (100) avec distribution automatique de fluide, qui comprend un réservoir de stockage (30') et une tête d'application (9') qui est disposée au-dessus du réservoir de stockage (30') et qui présente au moins un capteur (4) et une buse (5) pour la distribution de fluide, le capteur (4) étant relié fonctionnellement à une pompe (1) reliée à la buse (5) et raccordée au réservoir de stockage (30'), la tête d'application (9') présentant un bras d'application (15) sur lequel la buse (5) est disposée en étant dirigée vers le bas, et la pompe (1), qui peut être commandée par le capteur (4) et entraînée par moteur, étant disposée dans la tête d'application (9') avec une entrée et une sortie et reliée du côté de la sortie par une vanne (6), au moyen d'un raccord de tuyau ou de tube (5"), à une tubulure de liaison (5') de la buse (5) pour la distribution du fluide,
**caractérisé en ce qu'**un boîtier (9) et une pièce d'adaptation (8) sont prévus pour maintenir le réservoir de stockage, le boîtier (9) et la tête d'application (9') étant des composants séparés, la pièce d'adaptation (8) étant disposée sur la tête d'application (9') et un bord extérieur de la pièce d'adaptation (8') étant relié au boîtier (9) qui entoure au moins une partie d'un côté arrière du réservoir de stockage (30, 30'), le bras d'application (15) étant disposé sur le côté supérieur de la tête d'application (9'), et la pompe (1) étant disposée au-dessus de la pièce d'adaptation (8) au moyen de laquelle la tête d'application (9') peut être reliée de manière étanche aux fluides au réservoir de stockage (30').

5. Dispositif d'application de fluide (100) selon la revendication 4,
**caractérisé en ce que** la pièce d'adaptation (8) est disposée de manière permanente ou amovible sur la tête d'application (9'), une face inférieure de la pièce d'adaptation (8) pouvant être mise en prise avec une ouverture du réservoir de stockage (30'), et un dispositif de transport, en particulier un tuyau ou un tube (33), s'étendant du côté d'entrée de la pompe (1) dans le réservoir de stockage (30').

6. Dispositif d'application de fluide (100) selon la revendication 4 ou 5,
**caractérisé en ce que** la pièce d'adaptation (8) présente une face supérieure qui peut être reliée par complémentarité de forme à la tête d'application (9') et dont la face inférieure présente une bride, en particulier une bride (8') qui est décalée vers l'intérieur à partir d'un bord de la pièce d'adaptation et qui peut être mise en prise avec l'ouverture du réservoir de stockage (30'), et le dispositif de transport étant un tuyau de transport qui s'étend du côté d'entrée de la pompe (1) dans le réservoir de stockage (30') à travers une ouverture (18) prévue dans la pièce d'adaptation (8).

7. Dispositif d'application de fluide (90, 100) selon l'une des revendications précédentes,
**caractérisé en ce que** le capteur (4) ou les capteurs (4) est/sont en particulier un ou des capteur(s) optique(s) ou optoélectronique(s) (4), au moins un capteur (4) étant disposé sur le bras d'application (15) en étant dirigé vers le bas ou sur la tête d'application (9') en étant dirigé vers la buse (5).

8. Dispositif d'application de fluide (100) selon au moins l'une des revendications 4 à 7,
**caractérisé en ce que** la pompe pouvant être commandée par le ou les capteurs est une pompe à membrane qui est couplée fonctionnellement côté sortie à une vanne unidirectionnelle (6), la pompe à membrane étant couplée à une alimentation en énergie, en particulier à un magasin à piles (2) qui peut être remplacé dans son ensemble.

9. Dispositif d'application de fluide (90, 100) selon au moins l'une des revendications 1 à 8,
**caractérisé en ce que** l'unité de commande pour commander la vanne est également conçue comme une unité de régulation et est disposée en particulier sur une carte de circuit imprimé (3), un volume de distribution du fluide pouvant en particulier être prédéterminé.

10. Dispositif d'application de fluide (90, 100) selon l'une des revendications précédentes,
**caractérisé en ce que** dans la tête d'application (9') sont en outre disposés
- un dispositif pour mesurer la densité du fluide et/ou
- un dispositif pour détecter la nature de la main et/ou de la peau et/ou
- un dispositif pour déterminer la taille de la main, le dispositif pour déterminer la taille de la main étant couplé fonctionnellement au dispositif de commande et/ou de régulation.

11. Dispositif d'application de fluide (100) selon au moins l'une des revendications 1 à 3, ou 5 à 10, si dépendant de 1 à 3,
**caractérisé en ce qu'**un bord extérieur de la pièce d'adaptation (8') est relié à une pièce d'enveloppe (9) qui s'étend vers le bas à partir de la pièce d'adaptation (8) et qui entoure au moins une partie d'un côté arrière du réservoir de stockage (30, 30').

12. Dispositif d'application de fluide (90, 100) selon l'une des revendications précédentes,
**caractérisé en ce que** le boîtier (9) s'étend au moins jusqu'en dessous du réservoir de stockage (30, 30') et, en particulier au bord inférieur du boîtier (9), est disposé un bac collecteur (11) qui s'étend en dessous du bras d'application (15) selon une extension de ce dernier,
et/ou dans lequel le bac collecteur (11) présente un double fond, dont le fond supérieur (11') présente une pluralité d'ouvertures de passage (11") pour le fluide,
et dans lequel, de préférence, un dispositif d'aspiration, en particulier un rotor d'aspiration (12), est disposé dans le bac collecteur, en particulier sous le fond supérieur (11'), et en contact fluidique avec les ouvertures de passage (11") pour le fluide pour l'aspiration de fluide.

13. Dispositif d'application de fluide (90, 100) selon la revendication 12,
**caractérisé en ce qu'**au moins un moyen d'absorption de fluide est disposé dans le bac collecteur (11), en particulier sous le fond supérieur (11') qui présente la pluralité d'ouvertures de passage (11") pour le fluide, le moyen d'absorption étant en particulier un non-tissé absorbant ou un coussinet absorbant,
et/ou que le bac collecteur (11) est disposé de manière amovible sur le boîtier (9), et/ou qu'une traversée pour une ligne d'alimentation en énergie est prévue à un point de raccordement du bac collecteur (11) au boîtier (9).

14. Dispositif d'application de fluide (100) selon au moins l'une des revendications 4 à 13,
**caractérisé en ce que** la pièce d'adaptation (8) est conçue comme un écrou-raccord, est reliée à la tête d'application (9') et présente un filetage qui peut être vissé avec un filetage extérieur de col présent sur le réservoir de stockage (30'), et dans lequel le dispositif de transport est un tube de transport à double paroi (33), en particulier un tube de transport coaxial (33), dont le tube intérieur forme un magasin à piles (2) qui est relié électriquement au moins à la pompe (1), en particulier à la pompe et à un ou plusieurs consommateurs d'énergie présents dans la tête d'application (9') pour leur alimentation en énergie.

15. Dispositif d'application de fluide (90, 100) selon au moins l'une des revendications précédentes,
**caractérisé en ce que** le dispositif d'application de fluide (90, 100) est conçu
- pour une disposition sur pied ou
- pour une suspension murale, en particulier pour une suspension murale au moyen d'un support mural (10) disposé sur le boîtier (9) ou formé d'une seule pièce avec celui-ci, avec ou sans traversées électroniques, ou pour être disposé sur un meuble ou un autre bras de support vertical au moyen d'une pièce de serrage à vis (19) fixée au support mural (10).

16. Dispositif d'application de fluide (90, 100) selon au moins l'une des revendications précédentes,
**caractérisé en ce que** le boîtier (9) ou un support mural (10) formé d'une seule pièce avec celui-ci présente intérieurement un dispositif de levage (34) constitué d'une surface d'appui (34) qui est disposée de manière à pouvoir coulisser verticalement dans un élément de rail (34').

17. Dispositif d'application de fluide (90, 100) selon l'une des revendications précédentes,
**caractérisé en ce que** le boîtier (9) présente une partie avant (32) qui
- peut être relevée de manière articulée et à laquelle il est relié par une charnière reliée à son bord inférieur, ou qui
- peut être enfilée par le bas au moyen d'un rail.
